(19) Europäisches Patentamt
European Patent Office
Office européen des brevets

(11) **EP 1 330 221 B1**

(12) **EUROPEAN PATENT SPECIFICATION**

(45) Date of publication and mention
of the grant of the patent:
**22.05.2013 Bulletin 2013/21**

(21) Application number: **01973127.2**

(22) Date of filing: **19.09.2001**

(51) Int Cl.:
*A61K 9/00* (2006.01)　　　*A61K 9/70* (2006.01)
*A61K 47/18* (2006.01)　　　*A61K 45/06* (2006.01)
*A61F 13/00* (2006.01)　　　*A61P 17/02* (2006.01)
*A61P 31/02* (2006.01)

(86) International application number:
**PCT/US2001/029133**

(87) International publication number:
**WO 2002/024143 (28.03.2002 Gazette 2002/12)**

(54) **Medical compositions and dressings treating microbial infections of skin lesions**

Medizinische Zusammensetzungen und Verbände zur Behandlung von mikrobiellen Infektionen von Hautläsionen

Compositions pharmaceutiques et pansements permettant de traiter les infections microbiennes de lésions cutanées

(84) Designated Contracting States:
**AT BE CH CY DE DK ES FI FR GB GR IE IT LI LU MC NL PT SE TR**

(30) Priority: **20.09.2000　US 234375 P**

(43) Date of publication of application:
**30.07.2003　Bulletin 2003/31**

(73) Proprietor: **THE UNIVERSITY OF GEORGIA RESEARCH FOUNDATION, INC.**
**Athens, GA 30602-7411 (US)**

(72) Inventors:
 • **WOOLEY, Richard E.,**
**Dept. of Medical Microbiology**
**Athens, GA 30602-7386 (US)**
 • **RITCHIE, B.W.,**
**Dept. of Small Animal Medicine**
**Athens, GA 30602-7390 (US)**

(74) Representative: **OK pat AG**
**Chamerstrasse 50**
**6300 Zug (CH)**

(56) References cited:
　WO-A1-00/00186　　GB-A- 1 539 771
　US-A- 3 091 569　　US-A- 4 122 158
　US-A- 4 485 091　　US-A- 5 227 157
　US-A- 5 260 292　　US-B1- 6 270 781

 • FARCA A M ET AL: "POTENTIATION OF THE IN VITRO ACTIVITY OF SOME ANTIMICROBIAL AGENTS AGAINST SELECTED GRAM-NEGATIVE BACTERIA BY EDTA-TROMETHAMINE", VETERINARY RESEARCH COMMUNICATIONS, AMSTERDAM, NL, vol. 17, no. 2, 1 March 1993 (1993-03-01), pages 77-84, XP009068140, ISSN: 0165-7380, DOI: 10.1007/BF01839235
 • FARCA A M ET AL: "POTENTIATION OF ANTIBIOTIC ACTIVITY BY EDTA-TROMETHAMINE AGAINST THREE CLINICALLY ISOLATED GRAM-POSITIVE RESISTANT BACTERIA. AN IN VITRO INVESTIGATION", VETERINARY RESEARCH COMMUNICATIONS, AMSTERDAM, NL, vol. 18, no. 1, 1 January 1994 (1994-01-01), pages 1-06, XP009068138, ISSN: 0165-7380, DOI: 10.1007/BF01839255
 • GERBERICK G FRANKLIN ET AL: "IN VITRO SUSCEPTIBILITY OF PSEUDOMONAS AERUGINOSA TO CARBENICILLIN, GLYCINE, AND ETHYLENEDIAMINETETRAACETIC ACID COMBINATIONS", ANTIMICROBIAL AGENTS AND CHEMOTHERAPY, AMERICAN SOCIETY FOR MICROBIOLOGY, WASHINGTON, DC, US, vol. 17, no. 4, 1 April 1980 (1980-04-01), pages 732-735, XP001544096, ISSN: 0066-4804

EP 1 330 221 B1

## Description

### Field of the Invention

The present disclosure relates generally to pharmaceutical compositions, medical devices and ways of administration of the pharmaceutical compositions to inhibit microbial infections of surface wounds or skin lesions of a human or animal. More specifically, the present invention relates to medical dressings that may be applied to bums, ulcers or other lesions of the skin infected by microorganisms. The present disclosure further relates to washes to inhibit a microbial infection or colonization of the mouth and the skin surfaces, including those of the feet.

### Background

The outermost layers of the skin form a physical barrier that protects a human or animal from microbial invasion and the establishment of opportunistic infections. Injuries to the skin may be mechanical, such as an abrasion or cut, a burn from thermal, radiant or chemical exposure or a necrotic lesion of the surface tissue. A break in the cornified layer or epidermis allows microorganisms to penetrate deep into the lower tissues and establish an infection that can spread throughout the body. A burn injury or other skin surface lesion exposes tissue on which a microorganism can thrive. The extent of the infection will depend on the severity or depth of the burn and the surface area affected.

Bums are classified according to the degree of tissue damage. First degree bums are superficial and while the cornified layer may be breached, underlying tissue is not harmed. Second degree burns extend into the epidermal layer and trigger liquid loss with the possibility of shock. Some inhibition of the immune system may also occur. Third degree bums are considerably more severe and extend to the dermis or the underlying tissues. Charring of tissue is evident and there is severe disruption of the immune system.

The combination of a breach in the skin and a compromised immune system means that burn patients are highly susceptible to opportunistic infection of their wounds. Of the nearly two million burn victims per year in the United States, only about seventy thousand are serious enough to require hospitalization. However, as many as ten thousand of these patients will die, usually from a nosocomial infection. Many burn survivors will suffer permanent disfiguring, not just because of scar tissue formation, but also from the tissue damage that accompanies microbial infections. Greater treatment needs and prolongated care of bums patients in the hospital means that infections represent a significant financial drain. Long-term recuperative costs can also be significant.

Ulcers are exposed surface lesions of the skin or a mucoid layer such as the lining of the mouth, where inflamed and necrotic tissue sloughs off. The exposed surfaces of ulcers are highly susceptible to opportunistic microbial invasion. The primary infected site is localized and best treated by a topical application of an antimicrobial agent, perhaps augmented with systemic antibiotic administration. Although less dramatic in terms of morbidity and mortality than bums, infections of surface ulcers and other lesions of the skin and the mouth are nevertheless serious. The injured area is not usually as great as for a burn, but infected ulcers are discomforting to the patient or disfiguring, and even life-threatening if leading to a systemic infection.

Surface infections other than those resulting from physical injury or necrotic lesions of the skin or a mucosal surface are also known. Gingivitis, an infection of the gum of the mouth, dental caries, biofilm coverings on surfaces of teeth, and infections of the outer surface of the eye, for example, represent opportunistic microbial invasions requiring treatment with antimicrobial agents. Biofilms represent a particularly intransigent problem in that bacteria situated therein produce polysaccharide and mucoid coatings resistant to penetration by antibiotics.

Treatment of an infection of a burn or ulcer begins with mechanical cleaning of the wound, usually followed by covering with a bandage, gauze or similar dressing and disinfectant or antibiotic. Preferably the dressing will allow air circulation to prevent the establishment of an anaerobic bacterium and gangrene. Increasingly, however, preventive or active treatments of microbial, particularly nosocomial, infections of skin surface lesions encounter resistance of the infecting microorganism to most commonly available antibiotics and some disinfectants.

Microorganisms associated with nosocomial infections are normal flora in the human intestinal tract. Nosocomial infections, common in compromised patients including burn patients, have increased markedly in recent years. Bacteriemia in patients is especially due to *Staphylococcus. aureus, Pseudomonas aeruginosa, Escherichia coli,* or *Klebsiella sp.* and are particularly severe and difficult to treat when caused by methicillin-resistant *Staph. aureus,* vancomycin-resistant *Enterococcus sp.,* or *Ps. aeruginosa.* These organisms have been isolated from hospitals, chronic care facilities, and nursing homes. From 1991-1995, the number of bacteremias increased twofold as evidenced by the increased isolation of Enterococcal strains. An increase in incidents of antibiotic resistant strains of *Ps. aeruginosa, Enterobacter sp., Klebsiella sp.,* and *Enterococcus sp.,* has been reported. In particular, Staphylococcus has shown an alarming increase in resistance to oxacillin, and Enterococcus resistance to vancomycin. Bacteria are also becoming resistant to many chemical disinfectants, including quarternary ammonium ions due to the acquisition or activation of genes that encode for efflux pumps. The Arc MDR efflux pump, for example, protects bacteria from a wide spectrum of antibiotics, disinfectants, and organic solvents (*Alekshun & Levy*).

Gram-negative bacteria cell surfaces are damaged by the exposure to the chelating agent ethylenediaminetetraacetic acid (EDTA) *(Roberts et al.).* Tris buffer enhances this effect *(Goldschmidt & Wyss).* Gram-negative bacteria exposed to EDTA-Tris have increased permeability to extracellular solutes and leakage of intracellular solutes, periplasmic enzymes, cell membrane-associated proteins, lipopolysaccharide, protein, and phospholipids *(Bayer & Leive; Leive, L.).* They are also more sensitive to lysozyme, bactericides and antibiotics *(Brown & Richards; Monkhouse & Graves; Russel, A.D.; and Wooley & Jones).* The EDTA is a strong chelating agent that removes divalent cations from the bacterial cell wall *(Leive et al.),* altering the integrity and permeability of the outer membrane. Solutions of EDTA have also been shown to dissolve the biofilm or capsule produced by *Ps. aeruginosa (Kreig et al.).*

Combinations of EDTA-Tris solutions and selected antimicrobial agents have synergistic antimicrobial effects against *Ps. aeruginosa, E. coli* and *Proteus vulgaris (Farca et al.; Gerberick & Castric; Sparks et al.; Wooley & Jones; Wooley et al. (1983a); Wooley et al. (1984);* and *Wooley et al. (1983b,c)).* But, although combinations of EDTA-Tris with lysozymes or antibiotics are effective in the treatment of coliform infections of the urinary bladder in human patients *(Goldschmidt et al.),* bacterial rhinitis in dogs *(Wooley et al. (1979)),* induced *Pseudomonas* cystitis *(Wooley et al. (1974)),* otitis externa *(Blue et al.; Sparks et al.),* and multiple fistulas *(Ashworth et al; Bjorling et al.)* in dogs, and metritis in the mare *(Youngquist, RS.),* all of these treatments typically involve lavage treatment of infections.

Infections of burns, ulcers and other lesions, however, that have penetrated through the skin's cornified layer cannot be washed with lavages without a risk of promoting or spreading the infection, or leaving the wound open for further opportunistic infections. The volumes of lavages that would be used for seriously burned patients would also require large amounts of antibiotics, possibly encouraging antibiotic resistance in populations of microorganisms. Furthermore, seriously burnt patients may not tolerate extensive washing and manipulation of open and tender wound surfaces.

A method of administering an antimicrobial agent to a burn by incorporating the agent into a polymer that degrades and slowly releases the agent to the infected area is disclosed in Schmitt (U.S. Patent No. 4,122,158). This method, however, does not specifically address the need to enhance the effectiveness of an antibiotic to overcome antibiotic resistance. Yamasaki et al. in U.S. Patent No. 5,098,417 disclose cellulosic wound dressings with antibacterial and antifungal agents ionically bound thereon. The ionic bonds break when in contact with aqueous bodily exudates to release the biologically active compounds. The wound dressings of *Yamazaki et al.,* however, also do not address the need to enhance antibiotic activity to combat antibiotic resistant strains of bacteria. Raad et al. (U.S. Patent No. 5,688,516) discloses coating the exterior surface of a catheter device, inserted internally into a patient, with an antibiotic and a chelating agent to prevent infections that may be introduced into the patient by the catheter. There is still a need, however, not addressed in the prior art, for an enhanced antimicrobial composition effective in treating or preventing infections of burns and other skin or surface lesions of humans and animals. Safe and effective means of topically administering such compositions to a skin lesion, such as a burn, that will provide sustainable release of the antimicrobial agent to the wound are also required. The composition should preferably also have enhanced activity against drug resistant strains of infectious microbes.

**Summary of the Invention**

The scope of the invention is defined by the claims. Briefly described, the present invention provides a use of an antimicrobial composition in the manufacture of a medicament for inhibiting the proliferation of a microbial population of a skin injury or a surface lesion of a human or animal patient as defined in claim 1. The target is to inhibit an infection of a surface lesion of a human or animal, comprising and contacting a skin injury or surface lesion of a human or animal with an antimicrobial composition comprising a pharmaceutically acceptable antibiotic, a pharmaceutically acceptable chelating agent, and a pharmaceutically acceptable pH buffering agent, and vitamin E.

One aspect of the present invention is a medical dressing for delivering an antimicrobial composition to a site of a skin injury or lesion of a human or animal as defined in claim 17. The dressing comprises a support and an antimicrobial composition, wherein the antimicrobial composition comprises at least one pharmaceutically acceptable antibiotic having a concentration in the range of 0.04 mg/ml to 25.0 mg/ml and at least one pharmaceutically acceptable chelating agent selected from the group consisting **of** ethylenediaminetetraacetic acid (EDTA). trietbylene tetraamine dihydrochloride (TRIEN). ethylene glycol-bis (beta-aminoethyl ether).N,N,N1,N'-tetraacetic acid (EGTA), and diethylenetriaminpentaacetic acid (DPTA) and having a concentration in the range of 0.1 mM to 100 mM. The antimicrobial composition also includes an amount of tris (hydroxymethyl) aminomethane effective to maintain the pH of the composition in the range of 7.0 to 9.0 when in contact with the skin injury or lesion. This a pH buffering agent increases the effective activity of the antibiotic to a microbial infection.

In all embodiments of the present invention, the composition includes vitamin E that promotes tissue repair and reduces the pain associated with extensive burn injuries.

Also disclosed are methods for delivering an antibiotic to an injury to the skin of a human or animal, comprising the steps of identifying a site of microbial infection on the surface of a human or an animal, providing a medical dressing for delivering an antimicrobial composition to the site of infection, wherein the medical dressing comprises a support and an antimicrobial composition, the antimicrobial composition comprising at least one pharmaceutically acceptable antibiotic having antimicrobial activity, and at least one pharmaceutically acceptable chelating agent, pharmaceutically acceptable pH buffering agent as defined in claim 17, and vitamin E the medical dressing being intended to be applied to the site of infection.

Yet other aspects of the present invention relate to kits for the preparation of an antimicrobial composition to inhibit microbial infection of a skin injury or lesion of a human or animal as defined in claim 36. Another aspect of the present invention is a kit for the preparation of a medical dressing for the delivery of an antimicrobial composition to a site of infection as defined in claim 39. These kits comprise a medical dressing comprising a support, a first vessel containing at least one pharmaceutically acceptable chelating agent, at least one pharmaceutically acceptable antibiotic, at least one pharmaceutically acceptable pH buffering agent as defined in claim 17, vitamin E, and packaging material, with the packaging material, with the packaging material buffering agent as defined in claim 17, vitamin E, and packaging material, with the packaging material including instructions directing the use of the kit for preparing the medical dressing that is to be applied to a human or animal.

The present invention addresses the need for a medical dressing that can be applied to a wound to the skin, especially a burn injury, or a lesion such as an ulcer, and which will deliver a therapeutic amount of an antimicrobial composition that will be effective against infectious microorganisms. The present invention further addresses the need to deliver an effective amount of an antimicrobial composition that will overcome antibiotic resistance of many infectious organisms.

The present invention addresses these needs by providing medical dressings impregnated or having on a surface thereof, a composition comprising at least one antibiotic, a pharmaceutically acceptable pH buffering agent, at least one chelating agent that increases the sensitivity of a microorganism to the antibiotic, thereby increasing the efficacy of the antibiotic, and vitamin E. The present invention also addresses the need for a simple method of applying the medical dressing to a wound by supplying a kit containing the dressing and the composition that may be applied to the dressing before it is placed over a skin wound. The dressings and compositions of the present invention are also suitable for application to an injury to the mucosal surface of the mouth, or a tooth to treat or prevent a microbial infection thereof.

Additional objects, features, and advantages of the invention will become more apparent upon review of the detailed description set forth below when taken in conjunction with the accompanying drawing figures, which are briefly described as follows.

## Brief Description of the Figures

Figure 1. IsoboloGram, illustrating the combined effect of EDTA and neomycin (in 50 mM Tris) on *Staphylococcus aureus.*

Figure 2. IsoboloGram, illustrating the combined effect of EDTA and neomycin (in 50 mM Tris) on *Pseudomonas aeruginosa.*

Figure 3. IsoboloGram, illustrating the combined effect of EDTA and neomycin (in 50 mM Tris) on *Enterococcus faecalis.*

Figure 4. Growth of *Staphylococcus aureus* on Mueller Hinton agar when treated alone or with combinations of EDTA, water, and neomycin.

Figure 5. Growth of *Pseudomonas aeruginosa* on Mueller Hinton agar when treated alone or with combinations of EDTA, water, and neomycin.

Figure 6. Growth of *Enterococcus faecalis* on M Enterococcus agar when treated alone or with combinations of EDTA, water, and neomycin.

## Detailed Description of the Invention

A full and enabling disclosure of the present invention, including the best mode known to the inventor of carrying out the invention is set forth more particularly in the remainder of the specification, including reference to the Examples. This description is made for the purpose of illustrating the general principles of the invention.

The present invention addresses the need to deliver an effective dose of an antimicrobial composition of enhanced activity to a skin injury, such as a bum, a skin lesion or a lesion of the oral mucosa. The present invention provides medical dressings that are impregnated, or have on a surface thereof, a therapeutic antimicrobial composition comprising at least one antimicrobial agent a chelating agent, and a pharmaceutically acceptable pH buffering agent. The antimicrobial composition further comprises vitamin E that promotes tissue repair, thereby reducing the likelihood of further opportunistic infection and improved wound healing. The medical dressings of the present invention are useful for application to a burn or other traumatic injury to the skin of a human or animal, or to a skin lesion such as an ulcer or abrasion and which is or may become infected. The antimicrobial agent has increased antimicrobial activity because of the chelating agent and maintenance of the treated area at a pH suitable for sustained antibiotic activity. The antimicrobial agent can therefore be used in effective doses that are less than would be required for the same level of antimicrobial activity in the absence of the chelator. The compositions of the present invention are, therefore, useful in counteracting or preventing an infection or will be more effective against infections caused by drug-resistant strains of microbes.

The present invention further contemplates the use of a composition comprising an antimicrobial agent, a chelating agent and vitamin E in inhibiting a microbial infection or colonization of the oral mucosa. In one embodiment of the present invention the composition is in the form of a mouthwash or mouth rinse that may be contacted with the interior or exterior surfaces of a mouth or buccal cavity. In another embodiment of the present invention, the therapeutic antimicrobial composition impregnates or is on the surface of a medical dressing, including a gauze, a cloth, an absorbent or non-absorbent material that maybe placed onto a site of infection of the mouth. In another embodiment, the therapeutic antimicrobial composition may be mixed with, or otherwise combined with a malleable material such as a plastic, a resin or any other biologically acceptable material that can be inserted into the mouth for contacting an infected area within.

_Definitions_

The term "colonization" as used herein refers to the process of a group of bacteria living together. It is further understood that "colonization" may or may not result in a pathological infection.

The term "microbial infection" as used herein refers to any pathological presence of at least one bacterial species on or in an injury or lesion to the skin of a human or animal. It is further understood that a "microbial infection" may include any systemic infection that is amenable to inhibition by application of the antimicrobial compositions of the present invention.

The term "medical dressing" as used herein refers to any covering, protective or supportive, for diseased or injured parts of the skin, or internal organs of a human or animal. The dressing can bean absorbent dressing such as a gauze, a sterilized gauze or absorbent cotton, an antiseptic dressing permeated with an antiseptic solution to delay or prevent the onset of an infection, a dry dressing comprising a dry gauze, dry absorbent cotton or any other dry material that may be sterilized by any means known to one of ordinary skill in the art and which does not render the dressing unacceptable for placing over an open wound. The medical dressing as understood by the present invention may also comprise a non-adherent dressing that will not adhere to an infected wound or injury, a protective dressing intended to prevent further injury or infection to the infected part of the body, and a wet dressing wherein the dressing is wetted before application to the infected site. The term "medical dressing" further includes vitamin E in which an antimicrobial composition according to the present invention is dissolved. The oil-base vitamine E can form a barrier to further microbial infection and will leach an antimicrobial composition into the damaged tissue.

The term "bum" as used herein refers to tissue injury of the skin caused by thermal, chemical, or radiation exposure or abrasive friction. A burn may be a "first-degree burn" wherein there is superficial damage to the outer cornified layer, a "second-degree bum" wherein the damage extends down into the epidermal layer of cells but is not of sufficient extent that regeneration of the skin is prevented, or a "third-degree bum" wherein the injury extends below the dermis to the underlying tissue and wherein repair of the skin is not possible without grafting. The term "bum" as used herein also refers to any injury to the skin caused by an acid, an alkali, a brush or an abrasion, chemicals, electricity, explosive flash, hot liquids such as, but not only, boiling water, radiant energy such as heat, nuclear radiation or X-rays, or conductive thermal energy transfer due to direct contact with a hot surface or material. The term "burn" as used herein further refers to scalds due to exposure of the skin to hot liquids or gases that result in damage to the skin and underlying tissues.

The terms "lesion" and "surface lesion" as used herein refer to a circumscribed area of pathologically altered tissue, an injury or wound, or a single patch of a skin disease. The term "lesion" as used herein refers to primary lesions which are the immediate result of the pathological condition and may include cuts, abrasions, vesicles, blebs, bullae chancres, pustules, tubercles or any other such condition of the skin or a surface of the mouth, nose, anus or any other orifice of the body of a human or animal, or secondary lesions that later develop from a primary lesion and includes fissures and ulcers.

The term "ulcer" as used herein refers to an open sore or lesion of the skin or a mucous membrane that involves the sloughing off of inflamed and necrotized tissue and includes callous ulcers, chronic leg ulcers, decubitus, denture ulcers of the oral mucosa, traumatic ulcers of the mouth, infections stomatitis of the mouth and any type of secondary lesion that is a breach of the cornified and the epidermal layer of the skin.

The terms "antimicrobial composition", "antimicrobial agent" and "therapeutic antimicrobial composition" as used herein refer to the compounds and combinations thereof that may be administered to an animal or human and which inhibit the proliferation of a microbial infection.

The term "inhibiting the proliferation of a microbial population" as used herein refers to the bacteristatic or bacteriocidal activity of an antimicrobial composition. A bacterstatic antimicrobial composition will inhibit the multiplication of a bacterial population. A bacteriocidal antimicrobial composition will contact and kill the target microbial population.

The terms "mouthwash" and "mouth rinse" as used herein refer to a medicated solution used to treat or prevent diseases of the mouth or buccal cavity wherein the diseases compromise an infection by a microbial agent.

The term "impregnated" as used herein refers to wetting, soaking or saturating an absorbent material or medical dressing with a liquid. The absorbent material may be, but is not necessarily, dried before application of the dressing to a wound or lesion of the skin or other infected site.

The term "chelating agent" as used herein refers to any organic or inorganic compound that will bind to a metal ion having a valence greater than one, and includes organic chelating agents such as ethylenediamenetetracetic acid (EDTA), triethylene tetramine dihydrochloride (TRIEN), ethylene glycol-bis (beta-aminoethyl ether)-N, N, N', N'-tetracetic acid (EGTA), diethylenetriamin-pentaacetic acid (DPTA), and triethylenetetramine hexaacetic acid (TTG), deferoxamine, Dimercaprol, edetate calcium disodium, zinc citrate, penicilamine succimer and Editronate or any other pharmaceutically acceptable chelating agent, salt or combination thereof, known to one of ordinary skill in the art, and which will chelate divalent metal ions such as, but not only $Ca^{2+}$, $Mg^{2+}$, $Mn^{2+}$, $Fe^{2+}$, and $Zn^{2+}$.

The term "antibiotic" as used herein refers to any pharmaceutically acceptable compound known to one of ordinary skill in the art that will inhibit the growth of, or kill, bacteria. The term "antibiotic" includes β-lactams (penicillins and cephalosporins), vancomycins, bacitracins, macrolides (erythromycins), lincosamides (clindomycin), chloramphenicols, tetracyclines, aminoglycosides (gentamicins), amphotericns, cefazolins, clindamycins, mupirocins, sulfonamides and trimethoprim, rifampicins, metronidazoles, quinolones, novobiocins, polymixins and Gramicidins and any salts or variants thereof. It also understood that it is within the scope of the present invention that the tetracyclines include immunocycline, chlortetracycline, oxytetracycline, demeclocycline, methacycline, doxycycline and minocycline and the like. It is also further understood that it is within the scope of the present invention that aminoglycoside antibiotics includegentamicin, amikacin and neomycin.

The term "pH buffering agent" as used herein refers to any pharmaceutically acceptable organic or inorganic compound or combination of compounds that will maintain the pH of an antibiotic-containing solution within 0.5 pH units of a selected pH value. A "pH buffering agent" may be selected from, but is not limited to, Tris (hydroxymethyl) aminomethane (tromethaprim; TRIZMA base), or salts thereof, phosphates or any other buffering agent such as, for example, phosphate-buffered saline that is biologically acceptable.

The term "carrier" as used herein refers to any pharmaceutically acceptable solvent of antibiotics, chelating agents and pH buffering agents that will allow the antimicrobial composition of the present invention to be administered directly to a burn or other skin lesion or to the oral mucosa. The carrier will also allow the composition of the present invention to be applied to a medical dressing for application to the infected region of the skin or mouth of a human or animal. A "carrier" as used herein, therefore, refers to such solvent as, but not limited to, water, saline, physiological saline, ointments, creams, oil-water emulsions, gels, or any other solvent or combination of solvents and compounds known to one of skill in the art that is pharmaceutically and physiologically acceptable to the recipient human or animal. An example of a gel that may be used in compositions of the present invention is KY™ gel (sodium carboxymethylcellulose 7H 4F (Hercules Inc., Wilmington, DE)). An example of an oil-based carrier is vitamin E.

Microbial species that may cause infections inhibited by the present invention include bacterial species that may cause infections of a burn or lesion of the skin or oral mucosal lesion of a human or animal include *Aerobacter aerongenes, Aeromonas spp., Bacillus spp., Bordetella spp, Campylobacter spp., Chlamydia spp., Corynebacterium spp., Desulfovibrio spp., Escherichia coli, enteropathogenic E. coli, Enterotoxin-prodixing E coli, Helicobacter pylori, Klebsiella pneumoniae, Legionella pneumophiia, Leptospira spp., Mycobacterium tuberculosis, M bovis, Neisseria gonorrhoeae, N. meningitidis, Nocardia spp., Proteus mirabilis, P vulgaris, Pseudomonas aeruginosa, Rhodococcus equi, Salmonella enteridis, S. typhimurium, S. typhosa, Shigella sonnei, S dysenterae, Staphylococcus atreus, Staph. epidermidis, Streptococcus anginosus, S. mutans, Vibrio cholerae, Yersinia pestis, Y. pseiidotuberculosis, Actinomycetes spp.,* and *Streptomyces spp.*

The term "biologically active" as used herein refers to the ability of an antibiotic to inhibit the proliferation of a microorganism. The action of the antibiotic can be either bacteriostatic wherein the antibiotic arrests the proliferation of, but does not necessarily kill, the microorganism or the activity of the antibiotic can be bacteriocidal and kill the organism or a combination of activities. Techniques to identify the infecting microorganism and to determine the concentration of the antibiotic that will inhibit or kill fifty percent ($MIC_{50}$) of the organisms will be considered well known to one of ordinary skill in the art and will not require undue experimentation. The techniques to determine the antibiotic sensitivity of a bacterial isolate, and the methods of determining the synergistic effect of adding a chelating agent to a solution of an antibiotic are described in Manual of Methods for General Microbiology, Eds: Gerhardt et al., American Society of Microbiology, 1981. The term "pharmaceutically acceptable" as used herein refers to a compound or combination of compounds that will not damage the physiology of the recipient human or animal to the extent that the viability of the recipient is compromised. Preferably, the administered compound or combination of compounds will elicit, at most, a temporary detrimental effect on the health of the recipient human or animal.

The infection that may be treated by the medical dressings and compositions of the present invention, may be any opportunistic infection by a bacterium, or a multiple infection of more than one species of bacteria, and wherein the proliferation of which can be inhibited by the application of the compositions and medical dressings of the present invention.

The dressings of the present invention also include tissue growth promoting factors and vitamin E that are especially useful for treating nosocomial infections of burn patients, and for promoting the healing of the injuries so treated. Tissue growth and repair is promoted, thereby reducing final tissue damage and increasing the acceptance of graft tissue at the injured site, as shown in Example 6, below.

The therapeutic antimicrobial composition of the present invention further comprises vitamin E that is useful in reducing the production of toxic compounds at the injury site and for promoting tissue repair.

It is contemplated by the present invention that the antimicrobial composition may be used as a mouth wash or rinse to inhibit the proliferation of infections of lesions of the oral surfaces including the oral mucosa, the teeth, tongue or other surfaces of the oral cavity. The inclusion of a pharmaceutically acceptable chelating agent is useful in attacking biofilms on, for example, teeth that would otherwise prevent access of an antimicrobial agent to the underlying bacteria. It is further contemplated that the antimicrobial composition of the present invention may be used as a bath for the total or partial immersion of a human or animal for the treatment of skin infections or lesions of the skin such as for inhibiting an infection of a foot, or hand, of a human or animal.

Before application of the composition of the present invention, it is anticipated that the identity and the resistance of the infecting microorganism to the antibiotic may be determined by routine tests well known to one of ordinary skill in the art, including determining the MIC and FIC of antibiotics in the absence and/or presence of a chelating agent, and the amount of the antimicrobial composition may be adjusted accordingly so as to inhibit growth of the microorganism. The concentrations and amounts of the antimicrobial agent and chelating agent are adjusted to levels that are physiologically accepted by the exposed tissue of the injury or lesion and effective against the microbial infection of the skin injury or skin lesion.

According to the present invention, the concentration of the antibiotic is in the range of 0.04 mg/ml to 25.0 mg/ml and the concentration of the chelating agent in the carrier is in the range of 0.1 mM to 100.0 mM.

The present invention further provides a medical dressing comprising a support and a pharmaceutically acceptable antimicrobial composition thereon. The support may be any material that is biologically acceptable and suitable for placing over a wound such as a burn, or a surface lesion of the skin or the oral mucosa or teeth of the mouth. In exemplary embodiments of the present invention, the support may be a woven or non-woven fabric of synthetic or non-synthetic fibers, or any combination thereof. The present invention further contemplates using a support comprising a polymer foam, a natural or man-made sponge, a gel or a membrane that may absorb or have disposed thereon, a therapeutic composition. A gel suitable for use as a support for the antimicrobial composition of the present invention is KY™ (sodium carboxymethylcellulose 7H 4F (Hercules, Inc., Wilmington, DE)). The oily vitamin E is used as a solvent of an antimicrobial agent, chelating agent and optionally a pH buffering agent, and is used to cover a skin injury or lesion.

The present invention contemplates still further that the support may be a film, a natural or synthetic polymer, or a rigid or malleable material that is known to one of ordinary skill in the art as being acceptable for insertion in the mouth of a human or animal, and which will place an antimicrobial composition according to the present invention in contact with a tooth or a lesion of the oral mucosa. In one such embodiment of the medical dressing of the present invention, the support is a gauze. The gauze may be absorbent and can be wetted with an antimicrobial composition of the present invention before applying the gauze to an infected wound or other site.

The present invention also contemplates that the gauze may be impregnated with an antimicrobial composition and then dried. This allows the impregnated dressing to be stored for later use, or to avoid excessively dampening an injured area. In yet another embodiment of the present invention, an antimicrobial composition is absorbed on the surface of the support material of the medical dressing. The composition may be applied to the surface by wetting the surface with a solution of the antimicrobial composition and drying the support to deposit the composition thereon. A concentration of the antimicrobial composition that is effective against the proliferation of a microorganism may be attained when the dressing is wetted by fluids from the patient's injury.

One embodiment of the present invention, therefore, is a gauze impregnated with a pharmaceutically acceptable antimicrobial composition that comprises at least one antimicrobial agent, a chelating agent, vitamin E and a pH buffering agent. Impregnating the absorbent material and placing of the dressing onto a burn or lesion of the skin will allow the antimicrobial agent to be progressively administered to the site of injury. While not wishing to be bound by any one theory, the chelating agent is believed to remove divalent cations from the cell wall of a microbe such as a bacterium, as well as create pores in the bacterial wall. The presence of the chelating agent with the antibiotic increases the effective antimicrobial activity of a particular antibiotic dose.

The selected antibiotic can be effective against opportunistic pathogens infecting a burn, a skin lesion or the oral mucosa. While it is not the intention of the present invention to limit the antimicrobial agent, in various embodiments of the present invention the antimicrobial agent is an antibiotic selected from a β-lactam, an aminoglycoside, a vancomycin, a bacitracin, a macrolide, an erythromycin, a lincosamide, a chloramphenicol, a tetracycline, a gentamicin, an amphotericin, a cefazolin, a clindamycin, a mupirocin, a nalidixic acid, a sulfonamide and trimethoprim, a streptomycin, a rifampicin, a metronidazole, a quinolone, a novobiocin, a polymixin or a Gramicidin.

In one embodiment, the antibiotic is a penicillin, an aminoglycosides; a vancomycin, a chloramphenicol, an erythromycin, a tetracycline, gentamicin, nalidixic acids, or a streptomycin. In another embodiment the antibiotic is tetracycline. In another embodiment of the present invention, the antibiotic is neomycin. In another embodiment of the present invention, the antibiotic is arnikacin. In yet another embodiment, the antibiotic is gentamicin.

The pharmaceutically acceptable chelating agent of the antimicrobial composition applied to the medical dressing of the present invention is selected from ethylenediamenetetracetic acid (EDTA), triethylene tetramine dihydrochloride (TRIEN), ethylene glycol-bis (beta-aminoethyl ether)-N, N, N', N'-tetracetic acid (EGTA), and diethylenetriamin-pentaacetic acid (DPTA)

In other embodiments, the pharmaceutically acceptable chelating agent may be ethylenediamenetetracetic acid (EDTA) or triethylene tetramine dihydrochloride (TRIEN).

The antimicrobial compositions of the present invention further comprise a pharmaceutically acceptable pH buffering agent that will maintain the pH of the antimicrobial composition, when delivered to the skin injury or skin lesion, to between pH 7.0 and pH 9.0. In one embodiment of the present invention, the pH of the antimicrobial composition in solution is 8.0.

The antimicrobial composition of the present invention also includes vitamin E that will promote tissue growth and repair and reduces the pain experienced at the site of the skin injury. By promoting tissue repair, not only is discomfort to the patient reduced, but there may be less scar tissue formation and hence less permanent disfiguring of the patient. Furthermore, faster healing of a skin injury or lesion promoted by vitamin E is useful in reducing the likelihood of a nosocomial infection and the problems associated therewith.

Another aspect of the present invention is kits that comprise the antimicrobial compositions, or the components to prepare the antimicrobial compositions, and packaging that includes instructions on how to prepare and use the compositions to inhibit the proliferation of a microbial population of a skin injury such as a burn, or of a lesion of the skin or the oral mucosa and promote healing thereof. In one embodiment of the present invention, therefore, a kit for preparing the antimicrobial composition of the present invention for inhibiting the proliferation of a microbial infection comprises at least one vessel containing at least one pharmaceutically acceptable chelating agent, at least one pharmaceutically acceptable buffering agent suitable for maintaining the pH of the site of the infection in the range of 7.0 to 9.0 at least one pharmaceutically acceptable antimicrobial agent, vitamin E, and packaging material. The agent packaging material comprises instructions directing the use of the kit for preparing the antimicrobial composition of the present invention and delivering the composition to a skin injury or skin surface lesion, or to the mouth of a human or animal to inhibit the proliferation of a microbial infection therein.

Another embodiment of the present invention is a kit for the preparation of a medical dressing for the delivery of an antimicrobial composition according to the present invention to a site of a skin injury of a human or animal, comprising a medical dressing, at least one vessel containing at least one pharmaceutically acceptable chelating agent, at least one pharmaceutically acceptable antimicrobial agent, at least one pharmaceutically acceptable buffering agent, vitamin E and packaging material. The packaging material comprises instructions directing the use of the kit for preparing and applying the medical dressing to a human or animal to inhibit the growth of a microbial infection thereon.

The following examples are presented to describe the present invention.

### Example 1: Determination of synergistic actions and fractional inhibitory concentration (FIC) index

The antibacterial action of combinations of EDTA-Tris and neomycin was measured by a two-dimensional microtiter checkerboard technique described in Gilman et al., The Pharmacological Basis of Therapeutics, eds Goodman and Gilman, 1085-1086 (Macmillan Publishing Co., New York, 1985),. Sabath, L. D, Antimicrob. Agents and Chem. 210-217. (1967) and Sparks et al., Vet. Res. Comm.. 18, 241-249 (1994).

Each well of a round-bottomed 96-well microtiter plate was inoculated with 0.05 ml of 2-fold dilutions of neomycin, and of EDTA in 50 mM Tris. Then 0.05 ml of an 18-hour old culture of a test organism, containing $10^6$ colony-forming units (CFU)/ml, were added to each well. Controls for the culture and media were included in each plate. Plates were covered and incubated at 37°C for 18-24 hours.

Results were plotted as isobolograms for the determination of antagonistic, neutral or additive, or synergistic effects. To generate isobolograms, FICs of the two test solutions were plotted individually on the x-axis and y-axis to determine the effect of combining the two test solutions on bacterial growth. A line that curves away from the zero point and the coordinates indicates antagonism. A straight line indicates neutral or additive effects. Lines that curves toward the zero point and the coordinates are indicative of synergism if there is at least a 4-fold decrease in the MIC of each compound, when used in combination, as compared with the MIC of each test compound alone as described in *Gilman et al., The Pharmacological Basis of Therapeutics, eds Goodman and Gilman, 1085-1086 (Macmillan Publishing Co., New York, 1985),. Sabath, L. D, Antimicrob. Agents and Chem. 210-217. (1967).*

A numerical score or fractional inhibitory concentration (FIC) index was determined. The FIC index is equal to the sum of the values of FIC for the individual drugs:

$$FIC = \frac{\text{MIC of Drug A with Drug B}}{\text{MIC of Drug A}} + \frac{\text{MIC of Drug B with Drug A}}{\text{MIC of Drug B}}$$

An FIC index greater than 1.0 indicates an antagonistic interaction, an FIC index of 1.0 indicates addition, and an FIC index of less than or equal to 0.5 indicates synergism between the two test agents.

### Example 2: Inhibition of the growth of microorganisms infecting burns

The organisms of this study were isolated from human burn patients. They included strains of methicillin resistant *Staphylococcus aureus,* and vancomycin resistant strains of *Pseudomonas aeruginosa,* and *Enterococcus faecalis.* The bacterial isolates were propagated in or on Brain Heart Infusion broth (BHI), Mueller-Hinton Broth (MHB), blood agar (BA), Mueller-Hinton agar (MHA), enterococcus agar (EA), or 2X nutrient agar (2xNA).

The EDTA-Tris treatment solutions were prepared from a stock solution containing 0.5 mols/l sodium EDTA and 1.0 mols/l Tris-HCl, pH 8.0. The treatment solutions contained 5mM sodium EDTA and 50 mM Tris-HCl with or without of neomycin sulfate 1 mg / ml.

Antibiotic resistance profiles were determined by the disc diffusion method on MHA (5). Antibiotics tested included ampicillin (AM-10), chloramphenicol (C-30), ciprofloxacin (CIP-5), kanamycin (K-30), gentamicin (GM-10), nalidixic acid (NA-30), neomycin (N-30), streptomycin (S-10), sulfisoxazole (G-25), tetracycline (Te-30), and vancomycin (Va-30).

Minimal Inhibitory Concentrations (MICs) and Minimal Bactericidal Concentrations (MBCs) for EDTA-Tris and neomycin were determined by the broth-dilution microtiter method in MHB or BHI according to the method of Blair et al., Manual of Clinical Microbiology. p.307 (pub: Am. Soc. Microbiol. Williams and Wilkins, Baltimore 1970*).*

### Example 3: In vitro effect of EDTA-Tris and neomycin on *Enterococcus faecalis, Pseudomonas aeruginosa,* and *Staphylococcus aureus*

2xNA plates were swabbed with 200 ml of an overnight culture containing about $10^7$ colony-forming-units of a test organism. The plates were sampled with multipoint contactors as described in Wooley et al., Am. J. Vet. Res. 35, 807-810. (1974*).* Each multipoint contactor consisted of an array of 27mm sewing needles mounted to an aluminum plate measuring 1 mm x 50 mm x 50 mm. The needles were set 3.5 mm apart. The multipoint contactors were sterilized by autoclaving. To collect samples, a multipoint contactor was touched to an overnight bacterial culture grown on 2xNA as described above. Replicate plates were then inoculated by lightly pressing the needles bearing the test bacteria onto either MHA plates, BA plates or EA plates for *Ps. aeruginosa, Staph. aureus* and *Ent. faecalis* respectively. The agar plates were incubated at 37°C and colonies were counted at 24 hours and 48 hours.

Each strain of microorganism was tested on a control agar plate (plate 1), and on plates wherein the inoculated bacteria were covered with a sterile surgical gauze saturated with 7 ml of: 5 mM EDTA-Tris (plate 2); 5 mM EDTA-Tris and 1 mg/ml neomycin (plate 3); 1 mg/ml neomycin (plate 4); sterile water (plate 5). Samples were taken at 0 mins, and at 30 mins, 1 hour, 2 hours, 4 hours, 6 hours, 8 hours, and 24 hours of incubation.

### Example 4: The antibiotic resistance profiles, MIC and MBC values for test strains of *Staph. aureus, Ps. aeruginosa,* and *Ent. faecalis*

The antibiotic resistance profiles and MIC values for test strains of *Staph. aureus, Ps. aeruginosa,* and *Ent. faecalis* are shown on Table 1.

Table 1. Antibiotic resistance profiles of *Staphylococcus aureus, Pseudomonas aeruginosa,* and *Enterococcus faecalis.*

| | Antimicrobial Agents[A] | | | | | | | | | | |
|---|---|---|---|---|---|---|---|---|---|---|---|
| | Am | C | Cip | Gm | K | NA | N | S | G | Te | Va |
| *Staphylococcus aureus* | R[B] | I | R | S | R | R | R | S | S | S | S |
| *Pseudomonas aeruginosa* | R | R | I | I | R | R | R | R | R | R | R |
| *Enterococcus faecalis* | S | R | R | R | R | R | R | R | R | R | R |
| [A] Am= ampicillin; C= chloramphenicol; Cip= ciprofloxacin; K= kanamycin; Gm= gentamicin; NA= nalidixic acid; N= neomycin; S= streptomycin; G= sulfisoxazole; Te= tetracycline; Va= vancomycin; [B] R= resistant; I= intermediate; S= sensitive. | | | | | | | | | | | |

Fractional inhibitory concentrations (FICs) and isoboloGrams for the EDTA-Tris, neomycin combination to determine a synergistic, additive, or antagonistic reaction, as described in Example 1, were determined for *Staph. aureus, Ps. aeruginosa,* and *Ent. faecalis.* MIC and MBC values for concentrations of neomycin, ampicillin, chloramphenicol, amikacin and oxytetracycline and EDTA administered individually, and the FIC values for *Staph. aureus, Ps. aeruginosa,* and *Ent. faecalis* are shown in Table 2 (Columns 2 and 3). MIC values for mixtures of the above antibiotics and EDTA in the presence of each other are shown in Table 2 (Columns 4 and 5 respectively).

Table 2. Minimal Inhibitory Concentration (MIC) data concerning the amounts (mM) of EDTA in 50 mM Tris and antibiotics (mg/ml) when reacting alone and in combination against *Pseudomonas aeruginosa, Staphylococcus aureus,* and *Enterococcus faecalis.*

| | **MIC** | | | | |
|---|---|---|---|---|---|
| | Individually Administered | | Co-administered | | |
| | **Neomycin** | **EDTA** | **Neomycin + EDTA** | | **FIC** |
| *Ps. aeruginosa* | 1.0 | 1.25 | 0.063 | 0.156 | 0.19 |
| *Staph. aureus* | 3.13 | 1.0 | 1.56 | 0.25 | 0.75 |
| *Ent. faecalis* | 3.13 | 15.63 | 1.17 | 1.88 | 0.5 |
| | | | | | |
| | **Ampicillin** | **EDTA** | **Ampicillin + EDTA** | | **FIC** |
| *Ps. aeruginosa* | 0.49 | 1.25 | 0.123 | 0.156 | 0.38 |
| *Staph. aureus* | 0.24 | 1.0 | 0.0075 | 0.25 | 0.28 |
| *Ent. faecalis* | 0.001 | 15.63 | 0.00025 | 7.82 | 0.75 |
| | | | | | |
| | **Chloramphenicol** | **EDTA** | **Chloramphenicol + EDTA** | | **FIC** |
| *Ps. aeruginosa* | 12.5 | 1.25 | 1.56 | 0.313 | 0.37 |
| *Staph. aureus* | 0.39 | 1.0 | 0.39 | 1.0 | 2.0 |
| *Ent. faecalis* | 0.4 | 15.63 | 0.2 | 3.9 | 0.75 |

(continued)

| | Chloramphenicol | EDTA | Chloramphenicol + EDTA | | FIC |
|---|---|---|---|---|---|
| | | | | | |
| | Amikacin | EDTA | Amikacin + EDTA | | FIC |
| *Ps. aeruginosa* | 0.001 | 1.25 | 0.001 | 1.25 | 2.0 |
| *Staph. aureus* | 0.12 | 1.0 | 0.03 | 0.5 | 0.75 |
| *Ent. faecalis* | 2.0 | 15.63 | 1.0 | 7.8 | 1.0 |
| | | | | | |
| | Oxytetracycline | EDTA | Oxytetracycline + EDTA | | FIC |
| *Ps. aeruginosa* | 0.003 | 1.25 | 0.00075 | 0.313 | 0.5 |
| *Ent. faecalis* | 0.0001 | 1.0 | 0.00005 | 0.5 | 1.0 |
| *Staph. aureus* | 0.05 | 15.63 | 0.025 | 3.91 | 0.75 |
| * Synergistic reaction (FIC = $\leq 0.5$) Additive reaction (FIC = > 0.5 to $\leq 1.0$) Antagonistic reaction (FIC = > 1.0) | | | | | |

The MBC values for EDTA and neomycin were decreased by at least 75% for bacterial killing (MBC) in those situations in which synergistic potentation occurred (*Ps. aeruginosa* and *Ent. faecalis*) as shown in Table 3. A decrease of about 50% was observed with *Staph. aureus.*

Table 3. Minimal Bactericidal Concentrations (MBC), of *Staphylococcus aureus, Pseudomonas aeruginosa,* and *Enterococcus faecalis* reacted with EDTA (mM) and neomycin (mg/ml) in 50 mM Tris.

| Bacterial Species | | Individually Administered | Co-administered |
|---|---|---|---|
| *Staphylococcus aureus* | EDTA (mM) Neomycin (mg/ml) | 7.81 3.13 | 3.9 1.56 |
| *Pseudomonas aeruginosa* | EDTA (mM) Neomycin (mg/ml) | 250 5.0 | 20.0 0.04 |
| *Enterococcus faecalis* | EDTA (mM) Neomycin (mg/ml) | 250 25.0 | 62.5 6.25 |

Specifically in the case of *Staph. aureus,* the MBC values for EDTA and neomycin when combined were decreased by 50% as compared to the bactericidal effect of each when individually administered.

With *Ps. aeruginosa,* the MBC values for EDTA and neomycin when in combination were decreased 99.2% compared to when EDTA or neomycin were individually administered. In the case of *Ent. faecalis,* MBC values of EDTA and neomycin were both reduced 75% compared to when EDTA and neomycin were administered individually.

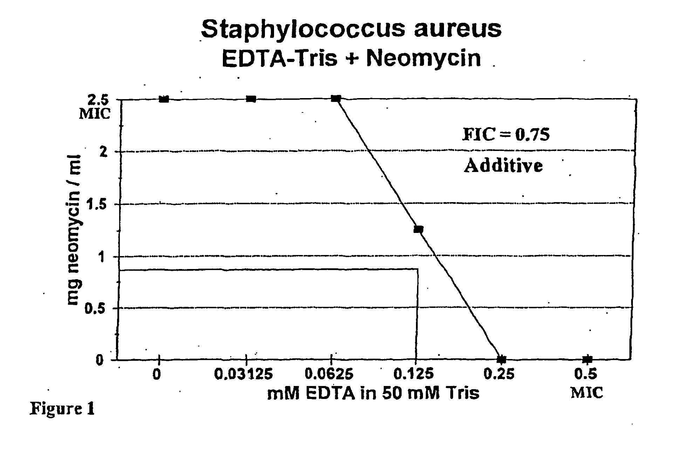

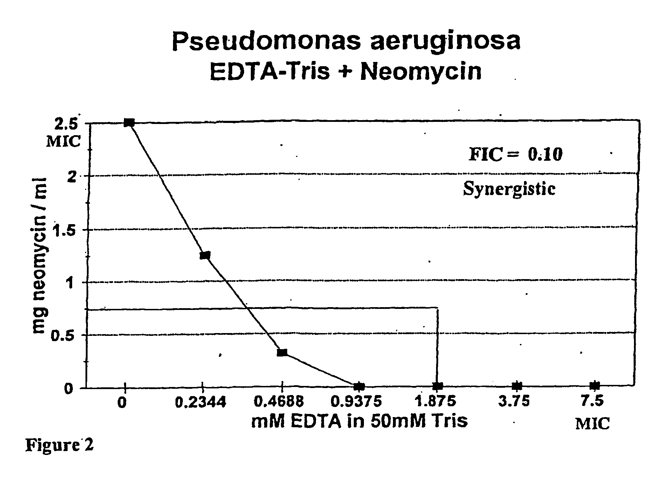

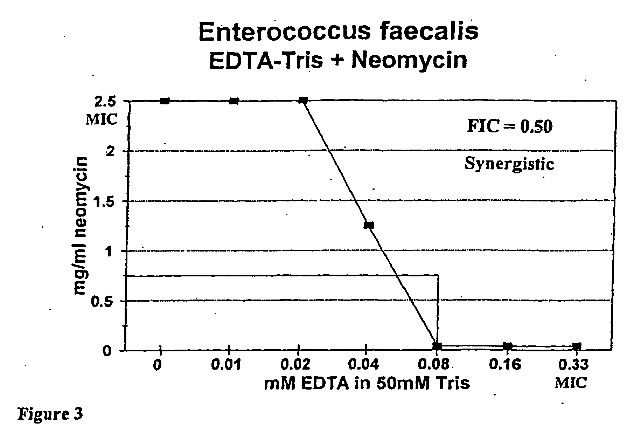

Synergistic effects were observed when various concentrations of EDTA-Tris and neomycin were reacted with *Ps. aeruginosa* and *Ent. faecalis,* while an additive effect was observed with *Staph. aureus* as shown in Figs. 1- 3.

## Example 5: Inhibition of growth of *Ps. aeruginosa* and *Staph. aureus*

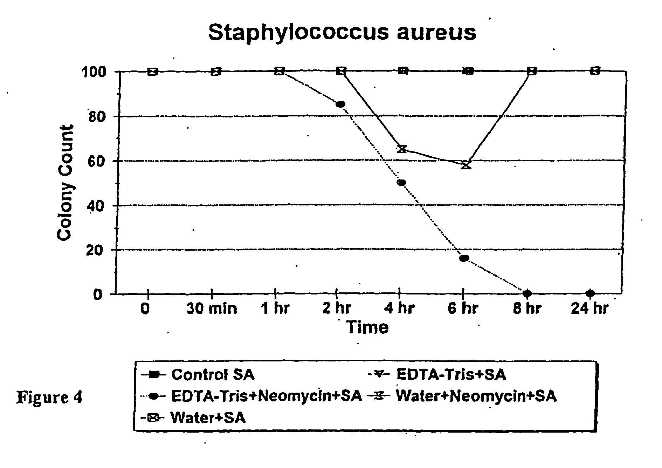

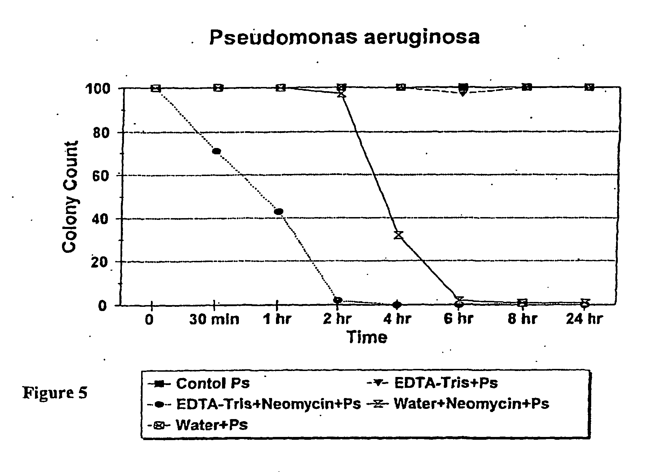

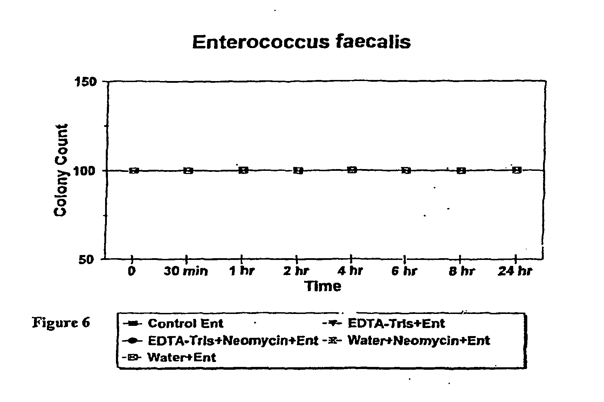

In the *in vitro* gauze-point-contactor study, the potentation effect was seen for EDTA-Tris-neomycin reactions with *Ps. aeruginosa* and *Staph aureus.* These reactions are illustrated in Figs. 4 and 5. When the same combinations of EDTA-Tris and neomycin were reacted with *Ent. faecalis,* no antibacterial activity was noted at these concentrations as shown in Fig. 6.

**Example 6: Treatment of a skin burn of a dog and antimicrobial protection of graft donor sites by vitamin E with EDTA-Tris and antibiotic**

A mixed-breed, 35 pound spayed female canine, 1-2 years old had been doused with gasoline and set on fire and severely burned. The dog was given initial emergency treatment for 5 days. The burned area over 30% of body was cultured for microbial infection and the following bacteria were identified: β-hemolytic *Escherichia coli, Klebsiella oxytoca, Proteus sp.,* and *Enterococcus sp.* The dog was administered cefazolin systemically and the burned area was cleared of tissue debris and wetted with a solution of EDTA-Tris and neomycin daily. The burn area was free of the four bacteria after 3 days of systemic and topical EDTA-Tris-neomycin therapy. After approximately 10 days, neomycin was replaced with amikacin. The dog received an autologous skin graft approximately three weeks after the burning incident and the donor site was treated with a compound consisting of EDTA-Tris-amikacin and 100 IU of vitamin E. The dog was discharged from veterinarian hospital care two weeks later.

**Example 7: Treatment of microbially infected skin and oral lesions**

A composition comprising EDTA, Tris and neomycin in KY™ gel carrier was applied to skin ulcers of a turtle, a snake and a frog. Infection was reduced until eliminated, and the treated animals fully healed of their injuries and infections.

A 13 year old domestic short hair cat had developed proliferative gingivitis. The mouth was swabbed with a cotton-tipped swab twice daily for a week with a solution containing 5 mM EDTA, 50 mM Tris, and 2 mg/ml neomycin. After the first week, the mouth and gums were swabbed twice weekly for a further month. Following clearance of the infection from the animal's mouth, there was no recurrence for at least one year. A similar human oral lesion also responded to this treatment. Likewise, mouthwashes also containing EDTA, Tris and neomycin, as above, were used to treat and heal infections stomatitis of the oral cavities of iguanas and snakes.

**Example 8: Vitamin E does not reduce the antibacterial efficacy of EDTA-Tris and antibiotics**

The addition of vitamin E to solutions of EDTA-Tris or EDTA-Tris plus antibiotics did not decrease the efficacy of the antibacterial action. The addition of vitamin E to solutions of EDTA-Tris, however, enhanced the antibacterial effect of the solution. Solutions of vitamin E alone decreased the numbers of *Ps. aeruginosa* significantly, as shown in Table 4.

Table 4: Effect of Vitamin E on the Efficacy of EDTA-Tris and Antibiotics when Tested against *Pseudomonas aeruginosa.*

| Groups | Log$_{10}$ Colony-Forming-Units/ml |
|---|---|
| PBS | 8.20 |
| EDTA-Tris | 3.30 |
| EDTA-Tris + Neomycin | No growth |
| EDTA-Tris + Amikacin | No growth |
| Vitamin E | 6.04 |
| Vit E + EDTA-Tris | No growth |
| Vit E + EDTA-Tris + Neomycin | No growth |
| Vit E + EDTA-Tris + Amikacin | No growth |
| Vit E + Neomycin | 4.40 |
| Vit E. + Amikacin | No growth |

**Example 9: Stability and Antibacterial Activity of EDTA-Tris-Neomycin solution (ETN) in KY gel**

Mixtures were prepared, stored at room temperature, and tested monthly against the test organism *Pseudomonas aeruginosa.* Compositions were prepared with or without KY™ gel. KY™ gel was Sodium Carboxymethylcellulose 7H 4F (Food Grade) (Hercules, Inc., Wilmington, DE).

Addition of 1% or 2% KY™ gel to the EDTA-Tris-antibiotic compositions did not affect the long term stability and antibacterial activity of the solutions of EDTA-Tris and neomycin, as shown in Table 5.

Table 5: Effect of mixing KY™ gel on EDTA-Tris-neomycin (ETN) storage stability.

| | Log $_{10}$ CFU/ml | | | |
|---|---|---|---|---|
| | PBS | ETN | ETN + 1 % KY gel | ETN + 2% KY gel |
| 1 month | 7.60 | NG | NG | NG |
| 2 months | 7.56 | NG | 4.04 | 4.48 |
| 3 months | 6.78 | NG | NG | 2.00 |
| 4 months | 7.73 | NG | NG | NG |
| 5 months | 6.64 | NG | NG | NG |
| 6 months | 6.94 | NG | NG | NG |
| 7 months | 7.40 | NG | NG | NG |
| 8 months | 7.56 | NG | NG | NG |
| 9 months | 8.00 | NG | NG | NG |
| 10 months | 7.85 | NG | NG | NG |
| NG = no growth | | | | |

**References**

The following references are specifically cited:

Alekshun, M.N. & Levy, S.B. Regulation of Chromosomally mediated Multiple Antibiotic Resistance: The mar Regulon. Antimicrob. Agents & Chemotherapy 41, 2067-2075 (1997).

Ashworth, C. D. & Nelson D. R. Antimicrob. Potentiation of Irrigation Solutions Containing Tris-(hydroxymethyl) aminomethane-EDTA. J. Am. Vet. Med. Assoc. 197, 1513-1514. (1990).

Bayer, M. E. & Leive L. Effect of Ethlyenediamintetraacetate Upon the Surface of Escherichia coli. J. Bacteriol. 130, (1364-1381. 1977).

Bjorling, D. E. &. Wooley R E. EDTA-Tromethamine Lavage as an Adjunct Treatment for Multiple Fistulas in a Dog. J. Am. Vet. Med. Assoc. 181, 596-597. (1982).

Blue, J. L., Wooley R. E. & Eagon, R. G. Treatment of Experimentally Induced Pseudomonas aeruginosa Otitis Externa in the Dog by Lavage with Ethylenediaminetetracetate-Tris (hydroxymethyl) Aminomethane Lysozyme. Am. J. Vet. Res. 35, 1221-1223. (1974).

Brown, M. R. W. & Richards, M. E. Effect of Ethylenediaminetetraacetate on the resistance of Pseudomonas aeruginosa to antibacterial agents. Nature (London). 207, 1391-1393. (1965).

Farca, A. M, Nebbia, P. & Re, G. Potentiation of the In Vitro Activity of Some Antimicrobial Agents against Selected Gram-Negative Bacteria by EDTA-Tromethamine. Vet. Res. Comm. 17, 77-84. (1993).

Gerberick, G. F. & Castric, P. A. In vitro Susceptibility of Pseudomonas aeruginosa to Carbenicillin, Glycine, and Ethylenediaminetetraacetic Acid Combinations. Antimicrob. Agents & Chemotherapy. 17, 732-735. (1980).

Goldschmidt, M C., Kuhn, C. R, Perry, K. & Johnson, D. E. EDTA and Lysozyme Lavage in the Treatment of Pseudomonas and Coliform Bladder Infections. J. Urol. 107, 969-972. (1972).

Goldschmidt, M C. & Wyse, O. The role of Tris in EDTA Toxicity and Lysozyme Lysis. J. Gen. Microbiol. 47, 421-431 (1967).

Kreig, D.P., Bass, A. & Mattingly, S.J. Phosphorylcholine stimulates Capsule Formation of Phosphate-Limited Mucoid Pseudomonas aeruginosa. Infect. Immun. 56, 864-873 1988).

Leive, L. A Nonspecific Increase in Permeability in Escherichia coli Produced by EDTA. Proc. Nat. Acad. Sci. USA.53, 745-750 (1968).

Leive, L., Shovlin, V. K. & Mergenhagen, S. E. Physical, Chemical, and Immunological Properties of Lipopolysaccharide Released from Escherichia coli by Ethylenediaminetetraacetate. Biol. Chem. 243, 6384-6391 (1968).

Monkhouse, D. C. & Graves, G. A. The Effect of EDTA on the Resistance of Pseudomonas aeruginosa to Benzalkonium, Chloride. Aust. J. Pharm. 48, 570-575 (1967).

Roberts, N. A., Gray, G. W. & Wilkinson, S. C. The Bactericidal Action of Ethylenediamine-tetra-acetlc Acid on Pseudomonas aeruginosa. Microbios 7-8, 189-208. (1970).

Russel, A. D. Effect of Magnesium Ions & Ethylenediaminetetraacetic acid on the Activity of Vancomycin against Escherichia coli and Staphylococcus aureus. J. Appl. Bacteriol. 30, 395-401 (1967).

Sabath, L. D. Synergy of Antibacterial Substances by Apparently Known Mechanisms. Antimicrob. Agents & Chemotherapy. 210-217 (1967).

Sparks, T. A., Kemp, D. T., Wooley R E. & Gibbs, P. S. Antimicrobial Effect of Combinations of EDTA-Tris and Amikacin or Neomycin on the Microorganisms Associated with Otitis Externa in Dogs. Vet. Res. Comm. 18, 241-249 (1994).

Wooley, R. E., Berman, A. P. & Shotts Jr, E. B. Antibiotic-Tromethamine-EDTA Lavage for the Treatment of Bacterial Rhinitis in a Dog. J. Am. Vet. Med. Assoc. 75, 817-818 (1979).

Wooley, R. E. & Blue, J. L. In Vitro Effect of EDTA-Tris-Lysozyme on Selected Pathogenic Bacteria. J. Med. Microbiol.8, 189-194 (1975).

Wooley, R. E., Blue, J. L., Scott, T. A. & Belcher, M K. Attempt to Induce Pseudomonas pyoderma in the Dog. Am. J. Vet. Res. 35, 807-810 (1974).

Wooley, R. E., Dickerson, H. W., Siramens, K. W., Shotts Jr., E. B. & Brown, J. Effect of EDTA-Tris on an Escherichia coli Isolate Containing R Plasmids. Vet. Microbiol. 12, 65-75 (1986).

Wooley, R. E. & Jones, M. S. Action of EDTA-Tris and Antimicrobial Agent Combinations on Selected Pathogenic Bacteria. Vet. Microbiol. 8, 271-280 (1983).

Wooley, R E., Jones, M. S. & Shotts Jr., E. B. Uptake of Antibiotics in Gram-negative Bacteria Exposed to EDTA-Tris. Vet. Microbiol. 10, 57-70 (1984).

Wooley, R E., Jones, M. S., Gilbert, J. P. & Shorts Jr., E. B. In Vitro Action of Combinations of Antimicrobial Agents and EDTA-Tromethamine on Escherichia coli. Am. J. Vet. Res. 44, 1154-1158 (1983a).

Wooley, R E., Jones, M. S., Gilbert, J. P. & Shotts Jr., E. B. In Vitro Action of Combinations of Antimicrobial Agents with EDTA-Tromethamine on Proteus vulgaris of Canine Origin. Am. J. Vet. Res. 45, 1451-1454 (1984).

Wooley, R. E., Jones, M. S., Gilbert J. P., & Shotts Jr., E. B. In Vitro Action of Combinations of Antimicrobial Agents and EDTA-Tromethamine on Pseudonionas aeruginosa. Am. J. Vet. Res. 44, 1521-1524 (1983b).

Wooley, R E., Jones, M. S., Gilbert J. P., & Shotts Jr., E. B. In Vitro Effect of Combinations of Antimicrobial Agents and EDTA-Tromethamine on certain Gram-positive Bacteria. Am. J. Vet. Res. 44, 2167-2169 (1983c).

Wooley, R E., Schall, W. D., Eagon, R. G. & Scott, A. A. S. Efficacy of EDTA-Tris-Lysozyme Lavage in the Treatment of Experimentally Induced Pseudomonas aeruginosa Cystitis in the Dog. Am. J. Vet. Res. 35, 27-29 (1974).

Youngquist, R.S. Pseudomonas metritis in a mare. Vet. Med./Small An. Clinician 70, 340-342 (1975).

Schmitt (U.S. Patent No. 4,122,158)

Yamazaki et al. (U.S. Patent No. 5,098,417)

Raad et al. (U.S. Patent No. 5,688,516)

**Claims**

1. A use of an antimicrobial composition in the manufacture of a medicament for inhibiting the proliferation of a microbial population of a skin injury or a surface lesion of a human or animal patient, the antimicrobial composition comprising:

    a) a pharmaceutically acceptable antimicrobial agent having a concentration in the range of 0.04 mg/ml to 25.0 mg/ml;
    b) a pharmaceutically acceptable chelating agent selected from the group consisting of ethylenediaminetetraacetic acid (EDTA), triethylene tetraamine dihydrochloride (TRIEN), ethylene glycol-bis (beta-aminoethyl ether)-N, N,N',N'-tetraacetic acid (EGTA), and diethylenetriaminpentaacetic acid (DPTA) and having a concentration in the carrier in the range of 0.1 mM to 100 mM;
    c) an amount of tris (hydroxymethyl) aminomethane effective to maintain the pH of the composition in the range of 7.0 to 9.0 when in contact with the skin injury or lesion;
    d) vitamin E; and
    e) a pharmaceutically acceptable carrier.

2. The use of Claim 1, wherein the pharmaceutically acceptable chelating agent is ethylenediaminetetraacetic acid (EDTA).

3. The use of Claim 1 or 2, wherein the pharmaceutically acceptable antimicrobial agent is an antibiotic selected from the group consisting of a β-lactam, an aminoglycoside, a vancomycin, a bacitracin, a macrolide, an erythromycin, a lincosamide, a chloramphenicol, a tetracycline, a gentamicin, an amphotericin, a cefazolin, a clindamycin, a mupirocin, a nalidixic acid, a sulfonamide and trimethoprim, a streptomycin, a rifampicin, a metronidazole, a quinolone, a novobiocin, a polymixin and a Gramicidin.

4. The use of Claim 3, wherein the pharmaceutically acceptable antimicrobial agent is further selected from the group consisting of a β-lactam, an aminoglycoside, a vancomycin, a chloramphenicol, an erythromycin, a tetracycline, gentamicin, nalidixic acid and a streptomycin.

5. The use of one of the Claims 1 to 4, wherein the pharmaceutically acceptable antimicrobial agent is oxytetracycline.

6. The use of one of the Claims 1 to 4, wherein the pharmaceutically acceptable antimicrobial agent is amikacin.

7. The use of one of the Claims 1 to 4, wherein the pharmaceutically acceptable antimicrobial agent is neomycin.

8. The use of one of the Claims 1 to 4, wherein the pharmaceutically acceptable antimicrobial agent is biologically active against a Gram-negative bacterial species.

9. The use of one of the Claims 1 to 4, wherein the pharmaceutically acceptable antimicrobial agent is biologically active against a Gram-positive bacterial species.

10. The use of one of the Claims 1 to 4, wherein the microbial population is a bacterial infection comprising at least one Gram-negative bacterial genus selected from the group consisting of *Aeromonas, Pseudomonas, Escherichia, Enterococcus, Yersinia, Vibrio, Flexibacter, Nocardia, Flavobacterium, Edwardsiella* and *Cytophagia.*

11. The use of one of the Claims 1 to 4, wherein the microbial population is a bacterial infection comprising at least one Gram-positive bacterial genus selected from the group consisting of *Bacillus, Staphylococcus, Streptococcus,* and *Mycobacterium.*

**12.** The use of one of the Claims 1 to 11, wherein the skin injury is a burn.

**13.** The use of one of the Claims 1 to 11, wherein the skin injury is an abrasion.

**14.** The use of one of the Claims 1 to 11, wherein the skin injury is an ulcer.

**15.** The use of one of the Claims 1 to 11, wherein the surface lesion is a lesion of the oral mucosa of a human or animal patient.

**16.** The use of one of the Claims 1 to 15, wherein the antimicrobial composition is a mouthwash for inhibiting the proliferation of a microbial population of the oral cavity of a human or animal.

**17.** A medical dressing for delivering an antimicrobial composition to a skin injury or lesion in a human or animal, the medical dressing comprising a support and an antimicrobial composition, the antimicrobial composition comprising:

a) at least one pharmaceutically acceptable antimicrobial agent having a concentration in the range of 0.04 mg/ml to 25.0 mg/ml;
b) at least one pharmaceutically acceptable chelating agent selected from the group consisting of ethylenediaminetetraacetic acid (EDTA), triethylene tetraamine dihydrochloride (TRIEN), ethylene glycol-bis (beta-aminoethyl ether)-N,N,N',N'-tetraacetic acid (EGTA), and diethylene-H triaminpentaacetic acid (DPTA) and having a concentration in a pharmaceutically acceptable carrier in the range of 0.1 mM to 100 mM;
c) an amount of tris (hydroxymethyl) aminomethane effective to maintain the pH of the composition in the range of 7.0 to 9.0 when in contact with the skin injury or lesion; and
d) vitamin E.

**18.** The medical dressing of Claim 17, wherein the support is sterile.

**19.** The medical dressing of Claim 17 or 18, wherein the support is selected from the group consisting of woven fabrics of naturally occurring fibers, woven fabrics of man-made fibers, non-woven fabrics of naturally occurring fibers, non-woven fabrics of man-made fibers, strands of naturally occurring fibers, strands of man-made fibers, interconnected strands of man-made fibers and interconnected strands of naturally occurring fibers, polymer foams, naturally occurring or synthetic sponges, a biologically acceptable gel, and a membrane.

**20.** The medical dressing of Claim 17 or 18, wherein the support is a gauze material.

**21.** The medical dressing of Claim 17 or 18, wherein the support is a physiologically acceptable gel.

**22.** The medical dressing of Claim 19, wherein the membrane is a monolayer or a laminate, and wherein the membrane is comprised of a non-synthetic material, a synthetic material, or a combination thereof.

**23.** The medical dressing of one of the Claims 17 to 22, wherein the antimicrobial composition impregnates the support.

**24.** The medical dressing of one of the Claims 17 to 22, wherein the antimicrobial composition forms at least one layer on at least one surface of the support.

**25.** The medical dressing of Claim 17, wherein the support is selected from the group consisting of a film, a moldable plastic or resin, gauze, and fabric, and wherein the support is suitable for contacting a surface lesion in the mouth of a human or animal.

**26.** The medical dressing of Claim 17, wherein the pharmaceutically acceptable chelating agent is ethylenediaminetetraacetic acid (EDTA).

**27.** The medical dressing of one of the Claims 17 to 26, wherein the pharmaceutically acceptable antimicrobial agent is an antibiotic selected from the group consisting of a $\beta$-lactam, an aminoglycoside, a vancomycin, a bacitracin, a macrolide, an erythromycin, a lincosamide, a chloramphenicol, a tetracycline, a gentamicin, an amphotericin, a cefazolin, a clindamycin, a mupirocin, a nalidixic acid, a sulfonamide and trimethoprim, a streptomycin, a rifampicin, a metronidazole, a quinolone, a novobiocin, a polymixin and a Gramicidin.

28. The medical dressing of Claim 17, wherein the pharmaceutically acceptable antimicrobial agent is further selected from the group consisting of a penicillin, an aminoglycoside; a vancomycin, a chloramphenicol, an erythromycin, a tetracycline, gentamicin, nalidixic acid, and a streptomycin.

29. The medical dressing of Claim 17, wherein the pharmaceutically acceptable antimicrobial agent is oxytetracycline.

30. The medical dressing of Claim 17, wherein the pharmaceutically acceptable antimicrobial agent is selected from neomycin, amikacin and gentamicin.

31. The medical dressing of Claim 17, wherein the pharmaceutically acceptable antimicrobial agent is neomycin.

32. The medical dressing of Claim 17, wherein the pharmaceutically acceptable antimicrobial agent is amikacin.

33. The medical dressing of Claim 17, wherein the pharmaceutically acceptable antimicrobial agent is biologically active against Gram-negative bacterial species.

34. The medical dressing of Claim 17, wherein the pharmaceutically acceptable antimicrobial agent is biologically active against a Gram-positive bacterial species.

35. The medical dressing of Claim 17, wherein the pharmaceutically acceptable pH buffering agent in solution further adjusts the antimicrobial composition to a pH of 8.0.

36. A kit for preparing an antimicrobial composition for inhibiting the proliferation of a microbial infection or microbial colonization of a skin injury or surface lesion, the kit comprising:

   packaging material comprising at least one vessel containing:

   a) at least one pharmaceutically acceptable antimicrobial agent having a concentration in the range of 0.04 mg/ml to 25.0 mg/ml;
   b) at least one pharmaceutically acceptable chelating agent selected from the group consisting of ethylenediaminetetraacetic acid (EDTA), triethylene tetraamine dihydrochloride (TRIEN), ethylene glycol-bis (beta-aminoethyl ether)-N,N,N',N'-tetraacetic acid (EGTA), and diethylene-triaminpentaacetic acid (DPTA) and having a concentration in a pharmaceutically acceptable carrier in the range of 0.1 mM to 100 mM;
   c) an amount of tris (hydroxymethyl) aminomethane effective to maintain the pH of the composition in the range of 7.0 to 9.0 when in contact with the skin injury or lesion; and
   d) vitamin E;

   and instructions directing the use of the kit for preparing an antimicrobial composition for inhibiting the proliferation of a microbial population, and optionally for promoting tissue repair, of a skin injury or surface lesion of a human or animal.

37. The kit according to Claim 36, further comprising instructions directing the use of the kit for applying the antimicrobial composition to a skin injury or surface lesion of a human or animal to inhibit the proliferation of a microbial infection thereof.

38. The kit as in Claim 36 or 37, wherein the instructions direct the use of the antimicrobial composition for inhibiting an infection of a lesion of the oral mucosa of a human or animal patient.

39. The kit according to one of the Claims 36 to 38, further comprising a medical dressing, and instructions directing the use of the kit for preparing and applying the antimicrobial composition to the medical dressing and delivering the medical dressing to a human or animal to inhibit the proliferation of a microbial infection.

**Patentansprüche**

1. Verwendung einer antimikrobiellen Zusammensetzung bei der Herstellung eines Medikaments zur Hemmung der Proliferation einer Mikrobenpopulation bei einer Hautschädigung oder einer Oberflächenläsion eines menschlichen oder tierischen Patienten, wobei die antimikrobielle Zusammensetzung Folgendes umfasst:

a) ein pharmazeutisch annehmbares antimikrobielles Mittel in einer Konzentration im Bereich von 0.04 mg/ml bis 25.0 mg/ml;

b) einen pharmazeutisch annehmbaren Chelatbildner, der aus der Gruppe ausgewählt wird, die aus Ethylendiamintetraessigsäure (EDTA), Triethylentetraamindihydrochlorid (TRIEN), Ethylenglycol-bis(beta-aminoethylether)-N,N,N',N'-tetraessigsäure (EGTA) und Diethylentriaminpentaessigsäure (DPTA) besteht, und der in dem Träger eine Konzentration im Bereich von 0.1 mM bis 100 mM aufweist;

c) eine Menge an Tris(hydroxymethyl)aminomethan, die geeignet ist, den pH der Zusammensetzung im Bereich von 7.0 bis 9.0 zu halten, wenn sie sich in Kontakt mit der Hautschädigung oder Läsion befindet;

d) Vitamin E; und

e) einen pharmazeutisch annehmbaren Träger.

2. Verwendung nach Anspruch 1, wobei der pharmazeutisch annehmbare Chelatbildner Ethylendiamintetraessigsäure (EDTA) ist.

3. Verwendung nach Anspruch 1 oder 2, wobei das pharmazeutisch annehmbare antimikrobielle Mittel ein Antibiotikum ist, das aus der Gruppe ausgewählt wird, die aus einem β-Lactam, einem Aminoglycosid, einem Vancomycin, einem Bacitracin, einem Macrolid, einem Erythromycin, einem Lincosamid, einem Chloramphenicol, einem Tetracyclin, einem Gentamicin, einem Amphotericin, einem Cefazolin, einem Clindamycin, einem Mupirocin, einer Nalidixinsäure, einem Sulfonamid und Trimethoprim, einem Streptomycin, einem Rifampicin, einem Metronidazol, einem Chinolon, einem Novobiocin, einem Polymixin und einem Gramicidin besteht.

4. Verwendung nach Anspruch 3, wobei das pharmazeutisch annehmbare antimikrobielle Mittel ferner aus der Gruppe ausgewählt wird, die aus einem β-Lactam, einem Aminoglycosid, einem Vancomycin, einem Chloramphenicol, einem Erythromycin, einem Tetracyclin, Gentamicin, Nalidixinsäure und einem Streptomycin besteht.

5. Verwendung nach einem der Ansprüche 1 bis 4, wobei das pharmazeutisch annehmbare antimikrobielle Mittel Oxytetracyclin ist.

6. Verwendung nach einem der Ansprüche 1 bis 4, wobei das pharmazeutisch annehmbare antimikrobielle Mittel Amikacin ist.

7. Verwendung nach einem der Ansprüche 1 bis 4, wobei das pharmazeutisch annehmbare antimikrobielle Mittel Neomycin ist.

8. Verwendung nach einem der Ansprüche 1 bis 4, wobei das pharmazeutisch annehmbare antimikrobielle Mittel gegen eine gramnegative Bakterienspezies biologisch aktiv ist.

9. Verwendung nach einem der Ansprüche 1 bis 4, wobei das pharmazeutisch annehmbare antimikrobielle Mittel gegen eine grampositive Bakterienspezies biologisch aktiv ist.

10. Verwendung nach einem der Ansprüche 1 bis 4, wobei die Mikrobenpopulation eine bakterielle Infektion ist, die mindestens eine gramnegative Bakteriengattung umfasst, die aus der Gruppe ausgewählt wird, die aus *Aeromonas, Pseudomonas, Escherichia, Enterococcus, Yersinia, Vibrio, Flexibacter, Nocardia, Flavobacterium, Edwardsiella* und *Cytophagia* besteht.

11. Verwendung nach einem der Ansprüche 1 bis 4, wobei die Mikrobenpopulation eine bakterielle Infektion ist, die mindestens eine grampositive Bakteriengattung umfasst, die aus der Gruppe ausgewählt wird, die aus *Bacillus, Staphylococcus, Streptococcus* und *Mycobacterium* besteht.

12. Verwendung nach einem der Ansprüche 1 bis 11, wobei die Hautschädigung eine Verbrennung ist.

13. Verwendung nach einem der Ansprüche 1 bis 11, wobei die Hautschädigung eine Abschürfung ist.

14. Verwendung nach einem der Ansprüche 1 bis 11, wobei die Hautschädigung ein Geschwür ist.

15. Verwendung nach einem der Ansprüche 1 bis 11, wobei die Oberflächenläsion eine Läsion der Mundschleimhaut eines menschlichen oder tierischen Patienten ist.

**16.** Verwendung nach einem der Ansprüche 1 bis 15, wobei die antimikrobielle Zusammensetzung eine Mundspülung zur Hemmung der Proliferation einer Mikrobenpopulation in der Mundhöhle eines Menschen oder eines Tieres ist.

**17.** Medizinischer Verband zur Abgabe einer antimikrobiellen Zusammensetzung an eine Hautschädigung oder Läsion bei einem Menschen oder Tier, wobei der medizinische Verband einen Träger und eine antimikrobielle Zusammensetzung umfasst, wobei die antimikrobielle Zusammensetzung Folgendes umfasst:

a) mindestens ein pharmazeutisch annehmbares antimikrobielles Mittel in einer Konzentration im Bereich von 0.04 mg/ml bis 25.0 mg/ml;
b) mindestens einen pharmazeutisch annehmbaren Chelatbildner, der aus der Gruppe ausgewählt ist, die aus Ethylendiamintetraessigsäure (EDTA), Triethylentetraamindihydrochlorid (TRIEN), Ethylenglycol-bis(beta-aminoethylether)-N,N,N',N'-tetraessigsäure (EGTA) und Diethylentriaminpentaessigsäure (DPTA) besteht, und der in einem pharmazeutisch annehmbaren Träger eine Konzentration im Bereich von 0.1 mM bis 100 mM aufweist;
c) eine Menge an Tris(hydroxymethyl)aminomethan, die geeignet ist, den pH der Zusammensetzung im Bereich von 7.0 bis 9.0 zu halten, wenn sie sich in Kontakt mit der Hautschädigung oder Läsion befindet; und
d) Vitamin E.

**18.** Medizinischer Verband nach Anspruch 17, wobei der Träger steril ist.

**19.** Medizinischer Verband nach Anspruch 17 oder 18, wobei der Träger aus der Gruppe ausgewählt ist, die aus Geweben aus natürlich vorkommenden Fasern, Geweben aus künstlichen Fasern, Faservliesen aus natürlich vorkommenden Fasern, Faservliesen aus künstlichen Fasern, Strängen aus natürlich vorkommenden Fasern, Strängen aus künstlichen Fasern, miteinander verbundenen Strängen aus künstlichen Fasern und miteinander verbundenen Strängen aus natürlich vorkommenden Fasern, Polymerschaumstoffen, natürlich vorkommenden oder synthetischen Schwämmen, einem biologisch annehmbaren Gel und einer Membran besteht.

**20.** Medizinischer Verband nach Anspruch 17 oder 18, wobei der Träger ein Gazematerial ist.

**21.** Medizinischer Verband nach Anspruch 17 oder 18, wobei der Träger ein physiologisch annehmbares Gel st.

**22.** Medizinischer Verband nach Anspruch 19, wobei die Membran eine Monoschicht oder ein Laminat ist und wobei die Membran aus einem nichtsynthetischen Material, einem synthetischen Material oder einer Kombination davon besteht.

**23.** Medizinischer Verband nach einem der Ansprüche 17 bis 22, wobei die antimikrobielle Zusammensetzung den Träger imprägniert.

**24.** Medizinischer Verband nach einem der Ansprüche 17 bis 22, wobei die antimikrobielle Zusammensetzung mindestens eine Schicht auf mindestens einer Oberfläche des Trägers bildet.

**25.** Medizinischer Verband nach Anspruch 17, wobei der Träger aus der Gruppe ausgewählt ist, die aus einem Film, einem formbaren Kunststoff oder Harz, Gaze und Gewebe besteht und wobei der Träger geeignet ist, in Kontakt mit einer Oberflächenläsion im Mund eines Menschen oder Tieres zu gelangen.

**26.** Medizinischer Verband nach Anspruch 17, wobei der pharmazeutisch annehmbare Chelatbildner Ethylendiamintetraessigsäure (EDTA) ist.

**27.** Medizinischer Verband nach einem der Ansprüche 17 bis 26, wobei das pharmazeutisch annehmbare antimikrobielle Mittel ein Antibiotikum ist, das aus der Gruppe ausgewählt ist, die aus einem β-Lactam, einem Aminoglycosid, einem Vancomycin, einem Bacitracin, einem Macrolid, einem Erythromycin, einem Lincosamid, einem Chloramphenicol, einem Tetracyclin, einem Gentamicin, einem Amphotericin, einem Cefazolin, einem Clindamycin, einem Mupirocin, einer Nalidixinsäure, einem Sulfonamid und Trimethoprim, einem Streptomycin, einem Rifampicin, einem Metronidazol, einem Chinolon, einem Novobiocin, einem Polymixin und einem Gramicidin besteht.

**28.** Medizinischer Verband nach Anspruch 17, wobei das pharmazeutisch annehmbare antimikrobielle Mittel ferner aus der Gruppe ausgewählt ist, die aus einem Penicillin, einem Aminoglycosid, einem Vancomycin, einem Chloramphenicol, einem Erythromycin, einem Tetracyclin, Gentamicin, Nalidixinsäure und Streptomycin besteht.

**29.** Medizinischer Verband nach Anspruch 17, wobei das pharmazeutisch annehmbare antimikrobielle Mittel Oxytetracyclin ist.

**30.** Medizinischer Verband nach Anspruch 17, wobei das pharmazeutisch annehmbare antimikrobielle Mittel aus Neomycin, Amikacin und Gentamicin gewählt ist.

**31.** Medizinischer Verband nach Anspruch 17, wobei das pharmazeutisch annehmbare antimikrobielle Mittel Neomycin ist.

**32.** Medizinischer Verband nach Anspruch 17, wobei das pharmazeutisch annehmbare antimikrobielle Mittel Amikacin ist.

**33.** Medizinischer Verband nach Anspruch 17, wobei das pharmazeutisch annehmbare antimikrobielle Mittel gegen eine gramnegative Bakterienspezies biologisch aktiv ist.

**34.** Medizinischer Verband nach Anspruch 17, wobei das pharmazeutisch annehmbare antimikrobielle Mittel gegen eine grampositive Bakterienspezies biologisch aktiv ist.

**35.** Medizinischer Verband nach Anspruch 17, wobei der pharmazeutisch annehmbare pH-Puffer in der Lösung ferner die antimikrobielle Zusammensetzung auf einen pH von 8.0 einstellt.

**36.** Kit zur Herstellung einer antimikrobiellen Zusammensetzung zur Hemmung der Proliferation einer mikrobiellen Infektion oder einer mikrobiellen Besiedelung einer Hautschädigung oder Oberflächenläsion, wobei das Kit Folgendes umfasst:

Verpackungsmaterial, umfassend mindestens einen Behälter, der Folgendes enthält:

a) mindestens ein pharmazeutisch annehmbares antimikrobielles Mittel in einer Konzentration im Bereich von 0.04 mg/ml bis 25.0 mg/ml;
b) mindestens einen pharmazeutisch annehmbaren Chelatbildner, der aus der Gruppe ausgewählt wird, die aus Ethylendiamintetraessigsäure (EDTA), Triethylentetraamindihydrochlorid (TRIEN), Ethylenglycol-bis(beta-aminoethylether)-N,N,N',N'-tetraessigsäure (EGTA) und Diethylentriaminpentaessigsäure (DPTA) besteht, und der in einem pharmazeutisch annehmbaren Träger eine Konzentration im Bereich von 0.1 mM bis 100 mM aufweist;
c) eine Menge an Tris(hydroxymethyl)aminomethan, die geeignet ist, den pH der Zusammensetzung im Bereich von 7.0 bis 9.0 zu halten, wenn sie sich in Kontakt mit der Hautschädigung oder Läsion befindet;
d) Vitamin E.;

und Anleitungen zur Verwendung des Kits zur Herstellung einer antimikrobiellen Zusammensetzung zur Hemmung der Proliferation einer Mikrobenpopulation und gegebenenfalls zur Förderung der Gewebereparatur bei einer Hautschädigung oder Oberflächenläsion bei einem Menschen oder Tier.

**37.** Kit nach Anspruch 36, ferner umfassend Anleitungen zur Verwendung des Kits, um die antimikrobielle Zusammensetzung bei einer Hautschädigung oder Oberflächenläsion bei einem Menschen oder Tier anzuwenden, um die Proliferation einer mikrobiellen Infektion derselben zu hemmen.

**38.** Kit nach Anspruch 36 oder 37, wobei die Anleitungen Hinweise zur Verwendung der antimikrobiellen Zusammensetzung geben, um eine Infektion einer Läsion der Mundschleimhaut eines menschlichen oder tierischen Patienten zu hemmen.

**39.** Kit nach einem der Ansprüche 36 bis 38, ferner umfassend einen medizinischen Verband und Anleitungen zur Verwendung des Kits, um die antimikrobielle Zusammensetzung zuzubereiten und auf den medizinischen Verband aufzutragen und den medizinischen Verband bei einem Menschen oder Tier anzubringen, um die Proliferation einer mikrobiellen Infektion zu hemmen.

**Revendications**

1.  Utilisation d'une composition antimicrobienne dans la fabrication d'un médicament destiné à inhiber la prolifération d'une population microbienne d'une lésion cutanée ou d'une lésion superficielle chez un homme ou un animal, la composition antimicrobienne comprenant:

    a) un agent antimicrobien pharmaceutiquement acceptable ayant une concentration comprise entre 0.04 mg/ml et 25.0 mg/ml;
    b) un agent chélateur pharmaceutiquement acceptable choisi à partir du groupe composé d'acide éthylènediaminetétraacétique (EDTA), de dihydrochlorure de triéthylènetétramine (TRIEN), d'acide éthylène glycol bis (bêta-aminoéthyl éther)-N,N,N',N'-tétraacétique (EGTA) et d'acide diéthylènetriaminpentaacétique (DPTA) et ayant une concentration dans le tampon comprise entre 0.1 mM et 100 mM;
    c) une quantité de tris(hydroxyméthyl)aminométhane efficace pour maintenir le pH de la composition dans une plage comprise entre 7.0 et 9.0 lorsqu'elle est au contact de la blessure ou de la lésion cutanée;
    d) de la vitamine E; et
    e) un tampon pharmaceutiquement acceptable.

2.  Utilisation de la revendication 1, dans laquelle l'agent chélateur pharmaceutiquement acceptable est de l'acide éthylènediaminetétraacétique (EDTA).

3.  Utilisation de la revendication 1 ou 2, dans laquelle l'agent antimicrobien pharmaceutiquement acceptable est un antibiotique choisi parmi le groupe composé d'un bêta-lactame, un aminoglycoside, une vancomycine, une bacitracine, un macrolide, une érythromycine, un lincosamide, un chloramphénicol, une tétracycline, une gentamicine, une amphotéricine, une céfazoline, une clindamycine, une mupirocine, un acide nalidixique, un sulfonamide et un triméthoprime, une streptomycine, une rifampicine, un métronidazole, un quinolone, une novobiocine, une polymixine et une gramicidine.

4.  Utilisation de la revendication 3, dans laquelle l'agent antimicrobien pharmaceutiquement acceptable est en outre choisi parmi le groupe composé d'un bêta-lactame, un aminoglycoside, une vancomycine, un chloramphénicol, une érythromycine, une tétracycline, une gentamycine, un acide nalidixique et une streptomycine.

5.  Utilisation de l'une des revendications 1 à 4, dans laquelle l'agent antimicrobien pharmaceutiquement acceptable est une oxytétracycline.

6.  Utilisation de l'une des revendications 1 à 4, dans laquelle l'agent antimicrobien pharmaceutiquement acceptable est une amikacine.

7.  Utilisation de l'une des revendications 1 à 4, dans laquelle l'agent antimicrobien pharmaceutiquement acceptable est une néomycine.

8.  Utilisation de l'une des revendications 1 à 4, dans laquelle l'agent antimicrobien pharmaceutiquement acceptable est biologiquement actif contre les espèces bactériennes à Gram-négatif.

9.  Utilisation de l'une des revendications 1 à 4, dans laquelle l'agent antimicrobien pharmaceutiquement acceptable est biologiquement actif contre les espèces bactériennes à Gram-positif.

10. Utilisation de l'une des revendications 1 à 4, dans laquelle la population microbienne est une infection bactérienne comprenant au moins un genre de bactéries à Gram-négatif choisi à partir du groupe composé de Aeromonas, *Pseudomonas, Escherichia, Enterococcus, Yersinia, Vibrio, Flexibacter, Nocardia, Flavobacterium, Edwardsiella* et *Cytophagia.*

11. Utilisation de l'une des revendications 1 à 4, dans laquelle la population microbienne est une infection bactérienne comprenant au moins un genre de bactéries à Gram-positif choisi à partir du groupe composé de *Bacillus, Staphylococcus, Streptococcus* et *Mycobacterium.*

12. Utilisation de l'une des revendications 1 à 11, dans laquelle la lésion cutanée est une brûlure.

13. Utilisation de l'une des revendications 1 à 11, dans laquelle la lésion cutanée est une écorchure.

**14.** Utilisation de l'une des revendications 1 à 11, dans laquelle la lésion cutanée est un ulcère.

**15.** Utilisation de l'une des revendications 1 à 11, dans laquelle la lésion superficielle est une lésion des muqueuses orales d'un homme ou d'un animal.

**16.** Utilisation de l'une des revendications 1 à 15, dans laquelle la composition antimicrobienne est un collutoire destiné à inhiber la prolifération d'une population microbienne de la cavité orale d'un homme ou d'un animal.

**17.** Pansement médical destiné à délivrer une composition antimicrobienne à une blessure ou lésion cutanée chez un homme ou un animal, le pansement médical comprenant un support et une composition antimicrobienne, la composition antimicrobienne comprenant:

a) au moins un agent antimicrobien pharmaceutiquement acceptable ayant une concentration comprise entre 0.04 mg/ml et 25.0 mg/ml;
b) au moins un agent chélateur pharmaceutiquement acceptable choisi à partir du groupe composé d'acide éthylènediaminetétraacétique (ED-TA), de dihydrochlorure de triéthylènetétramine (TRIEN), d'acide éthylène glycol bis(bêta-aminoéthyl éther)-N,N,N',N'-tétraacétique (EGTA) et d'acide diéthylènetriaminpentaacétique (DPTA) et ayant une concentration dans un tampon pharmaceutiquement acceptable comprise entre 0.1 mM et 100 mM ;
c) une quantité de tris(hydroxyméthyl)aminométhane efficace pour maintenir le pH de la composition dans une plage comprise entre 7.0 et 9.0 lorsqu'elle est au contact de la blessure ou de la lésion cutanée; et
d) de la vitamine E.

**18.** Pansement médical de la revendication 17, dans lequel le support est stérile.

**19.** Pansement médical de la revendication 17 ou 18, dans lequel le support est choisi parmi le groupe composé de tissus tissés de fibres naturelles, de tissus tissés de fibres chimiques, de tissus non tissés de fibres naturelles, de tissus non tissés de fibres chimiques, de brins interconnectés de fibres chimiques et de brins interconnectés de fibres naturelles, de mousses polymère, d'éponges naturelles ou synthétiques, d'un gel biologiquement acceptable et d'une membrane.

**20.** Pansement médical de la revendication 17 ou 18, dans lequel le support est une gaze.

**21.** Pansement médical de la revendication 17 ou 18, dans lequel le support est un gel physiologiquement acceptable.

**22.** Pansement médical de la revendication 19, dans lequel la membrane est une monocouche ou un stratifié et dans lequel la membrane est composée d'un matériau non synthétique, d'un matériau synthétique ou d'une combinaison de ces derniers.

**23.** Pansement médical de l'une des revendications 17 à 22, dans lequel la composition antimicrobienne imprègne le support.

**24.** Pansement médical de l'une des revendications 17 à 22, dans lequel la composition antimicrobienne forme au moins une couche sur au moins une surface du support.

**25.** Pansement médical de la revendication 17, dans lequel le support est choisi parmi le groupe constitué d'un film, d'un plastique ou d'une résine modelable, d'une gaze et d'un tissu, et dans lequel le support est adapté pour être au contact d'une lésion superficielle dans la bouche d'un homme ou d'un animal.

**26.** Pansement médical de la revendication 17, dans lequel l'agent chélateur pharmaceutiquement acceptable est un acide éthylènediaminetétraacétique (EDTA).

**27.** Pansement médical de l'une des revendications 17 à 26, dans lequel l'agent antimicrobien pharmaceutiquement acceptable est un antibiotique choisi parmi le groupe composé d'un bêta-lactame, un aminoglycoside, une vancomycine, une bacitracine, un macrolide, une érythromycine, un lincosamide, un chloramphénicol, une tétracycline, une gentamicine, une amphotéricine, une céfazoline, une clindamycine, une mupirocine, un acide nalidixique, un sulfonamide et un triméthoprime, une streptomycine, une rifampicine, un métronidazole, un quinolone, une novobiocine, une polymixine et une gramicidine.

**28.** Pansement médical de la revendication 17, dans lequel l'agent antimicrobien pharmaceutiquement acceptable est choisi parmi le groupe composé d'une pénicilline, d'un aminoglycoside, d'une vancomycine, d'un chloramphénicol, d'une érythromycine, d'une tétracycline, d'une gentamicine, d'un acide nalidixique et d'une streptomycine.

**29.** pansement médical de la revendication 17, dans lequel l'agent antimicrobien pharmaceutiquement acceptable est une oxytétracycline.

**30.** Pansement médical de la revendication 17, dans lequel l'agent antimicrobien pharmaceutiquement acceptable est choisi parmi la néomycine, l'amikacine et la gentamicine.

**31.** Pansement médical de la revendication 17, dans lequel l'agent antimicrobien pharmaceutiquement acceptable est une néomycine.

**32.** Pansement médical de la revendication 17, dans lequel l'agent antimicrobien pharmaceutiquement acceptable est une amikacine.

**33.** Pansement médical de la revendication 17, dans lequel l'agent antimicrobien pharmaceutiquement acceptable est biologiquement actif contre les espèces bactériennes à Gram-négatif.

**34.** Pansement médical de la revendication 17, dans lequel l'agent antimicrobien pharmaceutiquement acceptable est biologiquement actif contre les espèces bactériennes à Gram-positif.

**35.** Pansement médical de la revendication 17, dans lequel l'agent tampon pH pharmaceutiquement acceptable ajuste en outre la composition antimicrobienne à un pH de 8.0.

**36.** Kit de préparation d'une composition antimicrobienne destinée à inhiber la prolifération d'une infection microbienne ou d'une colonisation microbienne d'une lésion cutanée ou d'une lésion superficielle, le kit comprenant:

a) au moins un agent antimicrobien pharmaceutiquement acceptable ayant une concentration comprise entre 0.04 mg/ml et 25.0 mg/ml;
b) au moins un agent chélateur pharmaceutiquement acceptable choisi à partir du groupe composé d'acide éthylènediaminetétraacétique (ED-TA), de dihydrochlorure de triéthylènetétramine (TRIEN), d'acide éthylène glycol bis(bêta-aminoéthyl éther)-N,N,N',N'-tétraacétique (EGTA) et d'acide diéthylènetriaminpentaacétique (DPTA) et ayant une concentration dans un tampon pharmaceutiquement acceptable comprise entre 0.1 mM et 100 mM;
c) une quantité de tris(hydroxyméthyl)aminométhane efficace pour maintenir le pH de la composition dans une plage comprise entre 7.0 et 9.0 lorsqu'elle est au contact de la blessure ou de la lésion cutanée; et
d) de la vitamine E.

et des instructions indiquant l'utilisation du kit pour préparer une composition antimicrobienne destinée à inhiber la prolifération d'une population microbienne, et éventuellement à favoriser la cicatrisation des tissus d'un lésion cutanée ou superficielle chez un homme ou un animal.

**37.** Kit selon la revendication 36, comprenant en outre des instructions indiquant l'utilisation du kit pour appliquer la composition antimicrobienne à une lésion cutanée ou superficielle chez un homme ou un animal afin d'inhiber la prolifération d'une infection microbienne.

**38.** Kit selon la revendication 36 ou 37, dans lequel les instructions indiquent l'utilisation de la composition antimicrobienne pour inhiber l'infection d'une lésion des muqueuses orales chez un homme ou un animal.

**39.** Kit selon l'une des revendications 36 à 38, comprenant en outre un pansement médical et des instructions indiquant l'utilisation du kit pour préparer et appliquer la composition antimicrobienne sur le pansement médical et appliquer le pansement médical à un homme ou un animal en vue d'inhiber la prolifération d'une infection microbienne.

**Staphylococcus aureus**
**EDTA-Tris + Neomycin**

Figure 1

# Pseudomonas aeruginosa
## EDTA-Tris + Neomycin

Figure 2

Enterococcus faecalis
EDTA-Tris + Neomycin

FIC = 0.50

Synergistic

Figure 3

## Staphylococcus aureus

Figure 4

Legend:
- Control SA
- EDTA-Tris+SA
- EDTA-Tris+Neomycin+SA
- Water+Neomycin+SA
- Water+SA

Pseudomonas aeruginosa

Figure 5

Legend:
- Contol Ps
- EDTA-Tris+Ps
- EDTA-Tris+Neomycin+Ps
- Water+Neomycin+Ps
- Water+Ps

## Enterococcus faecalis

Figure 6

Legend:
- Control Ent
- EDTA-Tris+Ent
- EDTA-Tris+Neomycin+Ent
- Water+Neomycin+Ent
- Water+Ent

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4122158 A, Schmitt
- US 5098417 A, Yamasaki

- US 5688516 A, Raad


### Non-patent literature cited in the description

- Manual of Methods for General Microbiology. American Society of Microbiology, 1981
- **GILMAN et al.** The Pharmacological Basis of Therapeutics. Macmillan Publishing Co, 1985, 1085-1086
- **SABATH, L. D,.** *Antimicrob. Agents and Chem.,* 1967, 210-217
- **SPARKS et al.** *Vet. Res. Comm.,* 1994, vol. 18, 241-249
- **BLAIR et al.** Manual of Clinical Microbiology. Williams and Wilkins, 1970, 307
- **WOOLEY et al.** *Am. J. Vet. Res.,* 1974, vol. 35, 807-810
- **ALEKSHUN, M.N. ; LEVY, S.B.** Regulation of Chromosomally mediated Multiple Antibiotic Resistance: The mar Regulon. *Antimicrob. Agents & Chemotherapy,* 1997, vol. 41, 2067-2075
- **ASHWORTH, C. D. ; NELSON D. R.** Antimicrob. Potentiation of Irrigation Solutions Containing Tris-(hydroxymethyl) aminomethane-EDTA. *J. Am. Vet. Med. Assoc.,* 1990, vol. 197, 1513-1514
- **BAYER, M. E. ; LEIVE L.** Effect of Ethylenediamintetraacetate Upon the Surface of Escherichia coli. *J. Bacteriol.,* 1977, vol. 130, 1364-1381
- **BJORLING, D. E. ; WOOLEY R E.** EDTA-Tromethamine Lavage as an Adjunct Treatment for Multiple Fistulas in a Dog. *J. Am. Vet. Med. Assoc.,* 1982, vol. 181, 596-597
- **BLUE, J. L. ; WOOLEY R. E. ; EAGON, R. G.** Treatment of Experimentally Induced Pseudomonas aeruginosa Otitis Externa in the Dog by Lavage with Ethylenediaminetetracetate-Tris (hydroxymethyl) Aminomethane Lysozyme. *Am. J. Vet. Res.,* 1974, vol. 35, 1221-1223
- **BROWN, M. R. W. ; RICHARDS, M. E.** Effect of Ethylenediaminetetraacetate on the resistance of Pseudomonas aeruginosa to antibacterial agents. *Nature (London),* 1965, vol. 207, 1391-1393
- **FARCA, A. M ; NEBBIA, P. ; RE, G.** Potentiation of the In Vitro Activity of Some Antimicrobial Agents against Selected Gram-Negative Bacteria by EDTA-Tromethamine. *Vet. Res. Comm.,* 1993, vol. 17, 77-84

- **GERBERICK, G. F. ; CASTRIC, P. A.** In vitro Susceptibility of Pseudomonas aeruginosa to Carbenicillin, Glycine, and Ethylenediaminetetraacetic Acid Combinations. *Antimicrob. Agents & Chemotherapy,* 1980, vol. 17, 732-735
- **GOLDSCHMIDT, M C. ; KUHN, C. R ; PERRY, K. ; JOHNSON, D. E.** EDTA and Lysozyme Lavage in the Treatment of Pseudomonas and Coliform Bladder Infections. *J. Urol.,* 1972, vol. 107, 969-972
- **GOLDSCHMIDT, M C. ; WYSE, O.** The role of Tris in EDTA Toxicity and Lysozyme Lysis. *J. Gen. Microbiol.,* 1967, vol. 47, 421-431
- **KREIG, D.P. ; BASS, A. ; MATTINGLY, S.J.** Phosphorylcholine stimulates Capsule Formation of Phosphate-Limited Mucoid Pseudomonas aeruginosa. *Infect. Immun.,* 1988, vol. 56, 864-873
- **LEIVE, L.** A Nonspecific Increase in Permeability in Escherichia coli Produced by EDTA. *Proc. Nat. Acad. Sci. USA.,* 1968, vol. 53, 745-750
- **LEIVE, L. ; SHOVLIN, V. K. ; MERGENHAGEN, S. E.** Physical, Chemical, and Immunological Properties of Lipopolysaccharide Released from Escherichia coli by Ethylenediaminetetraacetate. *Biol. Chem.,* 1968, vol. 243, 6384-6391
- **MONKHOUSE, D. C. ; GRAVES, G. A.** The Effect of EDTA on the Resistance of Pseudomonas aeruginosa to Benzalkonium, Chloride. *Aust. J. Pharm.,* 1967, vol. 48, 570-575
- **ROBERTS, N. A. ; GRAY, G. W. ; WILKINSON, S. C.** The Bactericidal Action of Ethylenediamine-tetra-acetlc Acid on Pseudomonas aeruginosa. *Microbios,* 1970, vol. 7-8, 189-208
- **RUSSEL, A. D.** Effect of Magnesium Ions & Ethylenediaminetetraacetic acid on the Activity of Vancomycin against Escherichia coli and Staphylococcus aureus. *J. Appl. Bacteriol.,* 1967, vol. 30, 395-401
- **SABATH, L. D.** Synergy of Antibacterial Substances by Apparently Known Mechanisms. *Antimicrob. Agents & Chemotherapy,* 1967, 210-217

- **SPARKS, T. A. ; KEMP, D. T. ; WOOLEY R E. ; GIBBS, P. S.** Antimicrobial Effect of Combinations of EDTA-Tris and Amikacin or Neomycin on the Microorganisms Associated with Otitis Externa in Dogs. *Vet. Res. Comm.,* 1994, vol. 18, 241-249
- **WOOLEY, R. E. ; BERMAN, A. P. ; SHOTTS JR, E. B.** Antibiotic-Tromethamine-EDTA Lavage for the Treatment of Bacterial Rhinitis in a Dog. *J. Am. Vet. Med. Assoc.,* 1979, vol. 75, 817-818
- **WOOLEY, R. E. ; BLUE, J. L.** In Vitro Effect of EDTA-Tris-Lysozyme on Selected Pathogenic Bacteria. *J. Med. Microbiol.,* 1975, vol. 8, 189-194
- **WOOLEY, R. E. ; BLUE, J. L. ; SCOTT, T. A. ; BELCHER, M K.** Attempt to Induce Pseudomonas pyoderma in the Dog. *Am. J. Vet. Res.,* 1974, vol. 35, 807-810
- **WOOLEY, R. E. ; DICKERSON, H. W. ; SIRAMENS, K. W. ; SHOTTS JR., E. B. ; BROWN, J.** Effect of EDTA-Tris on an Escherichia coli Isolate Containing R Plasmids. *Vet. Microbiol.,* 1986, vol. 12, 65-75
- **WOOLEY, R. E. ; JONES, M. S.** Action of EDTA-Tris and Antimicrobial Agent Combinations on Selected Pathogenic Bacteria. *Vet. Microbiol.,* 1983, vol. 8, 271-280
- **WOOLEY, R E. ; JONES, M. S. ; SHOTTS JR., E. B.** Uptake of Antibiotics in Gram-negative Bacteria Exposed to EDTA-Tris. *Vet. Microbiol.,* 1984, vol. 10, 57-70
- **WOOLEY, R E. ; JONES, M. S. ; GILBERT, J. P ; SHORTS JR., E. B.** In Vitro Action of Combinations of Antimicrobial Agents and EDTA-Tromethamine on Escherichia coli. *Am. J. Vet. Res.,* 1983, vol. 44, 1154-1158
- **WOOLEY, R E. ; JONES, M. S. ; GILBERT, J. P ; SHOTTS JR., E. B.** In Vitro Action of Combinations of Antimicrobial Agents with EDTA-Tromethamine on Proteus vulgaris of Canine Origin. *Am. J. Vet. Res.,* 1984, vol. 45, 1451-1454
- **WOOLEY, R. E. ; JONES, M. S. ; GILBERT J. P. ; SHOTTS JR., E. B.** In Vitro Action of Combinations of Antimicrobial Agents and EDTA-Tromethamine on Pseudonionas aeruginosa. *Am. J. Vet. Res.,* 1983, vol. 44, 1521-1524
- **WOOLEY, R E. ; JONES, M. S. ; GILBERT J. P. ; SHOTTS JR., E. B.** In Vitro Effect of Combinations of Antimicrobial Agents and EDTA-Tromethamine on certain Gram-positive Bacteria. *Am. J. Vet. Res.,* 1983, vol. 44, 2167-2169
- **WOOLEY, R E. ; SCHALL, W. D. ; EAGON, R. G. ; SCOTT, A. A. S.** Efficacy of EDTA-Tris-Lysozyme Lavage in the Treatment of Experimentally Induced Pseudomonas aeruginosa Cystitis in the Dog. *Am. J. Vet. Res.,* 1974, vol. 35, 27-29
- **YOUNGQUIST, R.S.** Pseudomonas metritis in a mare. *Vet. Med./Small An. Clinician,* 1975, vol. 70, 340-342